(19) Europäisches Patentamt
European Patent Office
Office européen des brevets

(11) Veröffentlichungsnummer: **0 370 293 B1**

# (12) EUROPÄISCHE PATENTSCHRIFT

(45) Veröffentlichungstag der Patentschrift: **12.05.93**

(51) Int. Cl.[5]: **C07D 249/12**, A01N 43/653

(21) Anmeldenummer: **89120422.4**

(22) Anmeldetag: **04.11.89**

Die Akte enthält technische Angaben, die nach dem Eingang der Anmeldung eingereicht wurden und die nicht in dieser Patentschrift enthalten sind.

## (54) Substituierte Triazolinone.

(30) Priorität: **19.11.88 DE 3839206**

(43) Veröffentlichungstag der Anmeldung:
**30.05.90 Patentblatt 90/22**

(45) Bekanntmachung des Hinweises auf die Patenterteilung:
**12.05.93 Patentblatt 93/19**

(84) Benannte Vertragsstaaten:
**BE CH DE FR GB IT LI NL**

(56) Entgegenhaltungen:
**EP-A- 0 294 666**
**US-A- 4 082 765**

**PATENT ABSTRACTS OF JAPAN, Band 2, Nr. 24, 16. Februar 1989, Seite 4198 C 77; & JP-A-52 125 168 (NIPPON SODA K.K.) 20-10-1977**

(73) Patentinhaber: **BAYER AG**

**W-5090 Leverkusen 1 Bayerwerk(DE)**

(72) Erfinder: **Müller, Klaus-Helmut, Dr.**
**Bockhackstrasse 55**
**W-4000 Düsseldorf 13(DE)**
Erfinder: **Lindig, Markus, Dr.**
**Dahlienweg 16**
**W-4010 Hilden(DE)**
Erfinder: **Findeisen, Kurt, Dr.**
**In der Follmühle 10**
**W-5068 Odenthal 2(DE)**
Erfinder: **König, Klaus, Dr.**
**Zum Hahnenberg 40**
**W-5068 Odenthal(DE)**
Erfinder: **Lürssen, Klaus, Dr.**
**August-Kierspel-Strasse 145**
**W-5060 Bergisch Gladbach 2(DE)**
Erfinder: **Santel, Hans-Joachim, Dr.**
**Grünstrasse 9a**
**W-5090 Leverkusen 1(DE)**
Erfinder: **Schmidt, Robert R., Dr.**
**Im Waldwinkel 110**
**W-5060 Bergisch Gladbach 2(DE)**
Erfinder: **Strang, Harry, Dr.**
**Heiderweg 53**
**W-4000 Düsseldorf 31(DE)**

EP 0 370 293 B1

Anmerkung: Innerhalb von neun Monaten nach der Bekanntmachung des Hinweises auf die Erteilung des europäischen Patents kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

## Beschreibung

Die Erfindung betrifft neue substituierte Triazolinone, mehrere Verfahren zu ihrer Herstellung und ihre Verwendung als Herbizide.

Es ist bekannt, daß bestimmte substituierte Triazolinone wie beispielsweise die Verbindung 4-Amino-1-(N-isopropylcarbamoyl)-3-methylthio-(1H,4H)-1,2,4-triazolin-5-on oder die Verbindung 4-Amino-1-(N-propylcarbamoyl)-3-methylthio-(1H,4H)-1,2,4-triazolin-5-on oder die Verbindung 4-Amino-1-(N-butylcarbamoyl)-3-methylthio-(1H,4H)-1,2,4-triazolin-5-on oder die Verbindung 4-Amino-1-(N-cyclohexylcarbamoyl)-3-methylthio-(1H,4H)-1,2,4-triazolin-5-on (vgl. z. B. JP 52/125 168) herbizide Eigenschaften besitzen. Weitere Beispiele unter Hinweis auf herbizide Eigenschaften finden sich in der EP-A-29 46 66. Die herbizide Wirksamkeit dieser vorbekannten Verbindungen gegenüber Problemunkräutern ist jedoch ebenso wie ihre Verträglichkeit gegenüber wichtigen Kulturpflanzen nicht in allen Anwendungsgebieten völlig zufriedenstellend.

Weiterhin sind bestimmte substituierte Triazolinone, wie beispielsweise das 1-(N,N-Dimethylcarbamoyl)-3-phenyl-4-amino-1,2,4-triazolin-5-on bekannt (vgl. J. Heterocycl. Chem. 17, 1691 - 1696 [1980]; Org. Mass. Spectrom. 14, 369 - 378 [1979]).
Über eine Wirksamkeit dieser vorbekannten Triazolinone als Herbizide ist bisher nichts bekannt.

Es wurden neue substituierte Triazolinone der allgemeinen Formel (I),

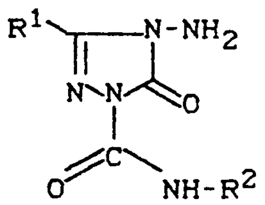

(I)

in welcher

$R^1$ für Alkyl mit 3 bis 4 Kohlenstoffatomen oder für Cyclopropyl steht und

$R^2$ für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl, Aryl, Aralkyloxy oder Aryloxy, für Alkoxy, Alkenyloxy oder Alkinyloxy steht,

gefunden.

Die Verbindungen der Formel (I) können gegebenenfalls in Abhängigkeit von der Art der Substituenten $R^1$ und $R^2$ als geometrische und/oder optische Isomere oder Isomerengemische unterschiedlicher Zusammensetzung vorliegen. Sowohl die reinen Isomeren als auch die Isomerengemische werden erfindungsgemäß beansprucht.

Weiterhin wurde gefunden, daß man die neuen substituierten Triazolinone der allgemeinen Formel (I),

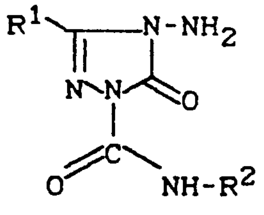

(I)

in welcher

$R^1$ für Alkyl mit 3 bis 4 Kohlenstoffatomen oder für Cyclopropyl steht und

$R^2$ für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls

substituiertes Aralkyl, Aroyl, Aryl, Aralkyloxy oder Aryloxy, für Alkoxy, Alkenyloxy oder Alkinyloxy steht,

erhält, wenn man

(a) Hydrazone der Formel (II),

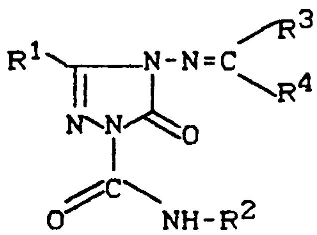

(II)

in welcher

$R^1$ und $R^2$ die oben angegebene Bedeutung haben und

$R^3$ und $R^4$ unabhängig voneinander jeweils für Wasserstoff, Alkyl, Aralkyl oder Aryl stehen,

mit einer Säure gegebenenfalls in Gegenwart eines Verdünnungsmittels umsetzt,

oder wenn man

(b) 1H-Triazolinone der Formel (III),

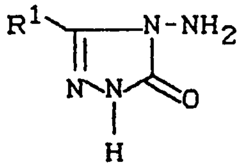

(III)

in welcher

$R^1$ die oben angegebene Bedeutung hat,

mit Isocyanaten der Formel (IV),

$$R^2 - N = C = O \quad (IV)$$

in welcher

$R^2$ die oben angegebene Bedeutung hat,

gegebenenfalls in Gegenwart eines Verdünnungsmittels und gegebenenfalls in Gegenwart eines Reaktionshilfsmittels umsetzt,

oder wenn man

(c) Triazolinone der Formel (V),

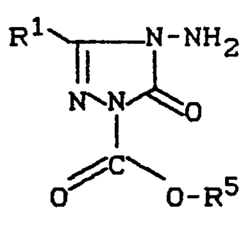

(V)

in welcher

$R^1$ die oben angegebene Bedeutung hat, und

$R^5$ für Alkyl, Aryl oder Arylalkyl steht,

mit Aminen der Formel (VI),

$$R^2 - NH_2 \quad (VI)$$

in welcher

$R^2$ die oben angegebene Bedeutung hat,
gegebenenfalls in Gegenwart eines Verdünnungsmittels und gegebenenfalls in Gegenwart eines Reaktionshilfsmittels umsetzt,
oder wenn man
(d) 1H-Triazolinone der Formel (III),

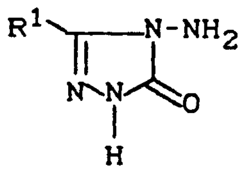

(III)

in welcher
$R^1$ die oben angegebene Bedeutung hat,
mit Urethanen der Formel (VII),

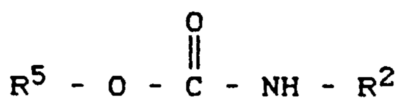

(VII)

in welcher
$R^2$ die oben angegebene Bedeutung hat und
$R^5$ für Alkyl, Aryl oder Arylalkyl steht,
gegebenenfalls in Gegenwart eines Verdünnungsmittels und gegebenenfalls in Gegenwart eines Reaktionshilfsmittels umsetzt.

Schließlich wurde gefunden, daß die neuen substituierten Triazolinone der allgemeinen Formel (I) herbizide Eigenschaften besitzen.

Überraschenderweise zeigen die erfindungsgemäßen substituierten Triazolinone der allgemeinen Formel (I) eine erheblich höhere herbizide Potenz gegenüber Problemunkräutern als die aus dem Stand der Technik bekannten substituierten Triazolinone, wie beispielsweise das 4-Amino-1-(N-isopropylcarbamoyl)-3-methylthio-(1H,4H)-1,2,4-triazolin-5-on oder die Verbindung 4-Amino-1-(N-propylcarbamoyl)-3-methylthio-(1H,4H)-1,2,4-triazolin-5-on oder die Verbindung 4-Amino-1-(N-butylcarbamoyl)-3-methylthio-(1H,4H)-1,2,4-triazolin-5-on oder die Verbindung 4-Amino-1-(N-cyclohexylcarbamoyl)-3-methylthio-(1H,4H)-1,2,4-triazolin-5-on, welche chemisch und wirkungsmäßig naheliegende Verbindungen sind.

Die erfindungsgemäßen substituierten Triazolinone sind durch die Formel (I) allgemein definiert. Bevorzugt sind Verbindungen der Formel (I), bei welchen

$R^1$ für n-Propyl, i-Propyl, n-Butyl, i-Butyl, s-Butyl, t-Butyl oder Cyclopropyl steht und

$R^2$ für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl oder Alkendiyl mit jeweils bis zu 4 Kohlenstoffatomen;

$R^2$ außerdem für im Heterocyclylteil gegebenenfalls einfach oder mehrfach, gleich oder verschieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen – insbesondere Stickstoff, Sauerstoff und/oder Schwefel – im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter $-O-CH_2-O-$ Gruppe steht.

Besonders bevorzugt sind Verbindungen der Formel (I), bei welchen

$R^1$ für n-Propyl, i-Propyl, i-Butyl, s-Butyl, t-Butyl oder Cyclopropyl steht und

$R^2$ Wasserstoff, Methyl, Ethyl, n- oder i-Propyl, n-, i-, s- oder t-Butyl, jeweils geradkettiges oder verzweigtes Pentyl, Hexyl, Heptyl, Octyl, Nonyl, Decyl, Dodecyl, für Allyl, jeweils geradkettiges oder verzweigtes Butenyl, Pentenyl oder Hexenyl, Propargyl, jeweils geradkettiges oder verzweigtes Butinyl, Pentinyl oder Hexinyl, für geradkettiges oder verzweigtes Halogenalkyl mit 1 bis 8 Kohlenstoffatomen und 1 bis 9 gleichen oder verschiedenen Halogenatomen, insbesondere Fluor, Chlor oder Brom, für jeweils geradkettiges oder verzweigtes Halogenalkenyl oder Halogenalkinyl mit jeweils 3 bis 8 Kohlenstoffatomen und 1 bis 3 Halogenatomen, insbesondere Fluor oder Chlor, für jeweils geradkettiges oder verzweigtes Cyanalkyl mit 1 bis 6 Kohlenstoffatomen im Alkylteil, Hydroxyalkyl mit 1 bis 6 Kohlenstoffatomen und 1 bis 3 Hydroxygruppen, Alkoxyalkyl, Alkoxycarbonylalkyl oder Alkoxycarbonylalkenyl, Alkylaminoalkyl oder Dialkylaminoalkyl mit jeweils bis zu 4 Kohlenstoffatomen in den einzelnen Alkyl- bzw. Alkenylteilen oder für jeweils gegebenenfalls ein- bis dreifach, gleich oder verschieden substituiertes Cyclopropyl, Cyclopropylmethyl, Cyclopropylethyl, Cyclopentyl, Cyclohexyl, Cycloheptyl, Cyclooctyl, Cyclohexylmethyl, Cyclohexylethyl, Cyclohexenyl oder Cyclohexenylmethyl steht, wobei als Substituenten jeweils in Frage kommen: Fluor, Chlor, Brom, Methyl, Ethyl, n- oder i-Propyl, n-, i-, s- oder t-Butyl, Cyano, Methandiyl, Ethandiyl, Butandiyl oder Butadiendiyl oder Dichlorallyl;

$R^2$ weiterhin für im Heterocyclylteil gegebenenfalls ein- bis dreifach, gleich oder verschieden substituiertes Heterocyclylmethyl, Heterocyclylpropyl oder Heterocyclylethyl steht, wobei als Heterocyclen jeweils in Frage kommen:

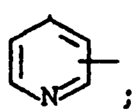 ; 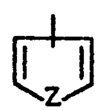 ; 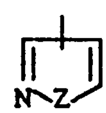 ; 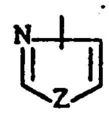 ;

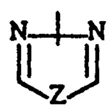 ; 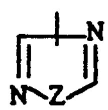 ; 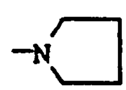 ; 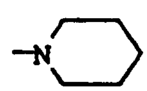 ;

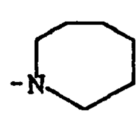 ; 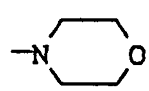 oder 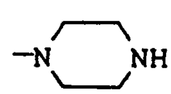

wobei Z jeweils für Sauerstoff oder Schwefel steht und wobei als Substituenten jeweils in Frage kommen:

Fluor, Chlor, Brom, Cyano, Nitro, Methyl, Ethyl, n- oder i-Propyl, n-, i-, s- oder t-Butyl, Methoxy, Ethoxy, Methylthio, Trifluormethyl, Trifluormethoxy oder Trifluormethylthio;

$R^2$ außerdem für jeweils geradkettiges oder verzweigtes Alkoxy mit 1 bis 6 Kohlenstoffatomen, Alkenyloxy mit 3 bis 6 Kohlenstoffatomen oder Alkinyloxy mit 3 bis 6 Kohlenstoffatomen oder für jeweils gegebenenfalls ein- bis dreifach, gleich oder verschieden substituiertes, gegebenenfalls geradkettiges oder verzweigtes Benzyl, Phenylethyl, Phenylpropyl, Phenylbutyl, Phenylpentyl, Phenylhexyl, Phenylheptyl, Phenylcyanmethyl, Phenylcyanethyl, Phenylcyanpropyl, Benzyloxy, Phenylethyloxy, Phenoxy, Benzoyl, Phenyl oder Naphthyl steht, wobei als Phenylsubstituenten jeweils in Frage kommen: Fluor, Chlor, Brom, Hydroxy, Cyano, Nitro, Methyl, Ethyl, n- oder i-Propyl, n-, i-, s- oder t-Butyl, Methoxy, Ethoxy, Methylthio, Trifluormethyl, Trifluormethoxy, Trifluormethylthio, Trifluormethylsulfinyl, Trifluormethylsulfonyl, Methylsulfinyl, Methylsulfonyl, Acetyl, Propionyl, Methoxycarbonyl, Ethoxycarbonyl, Cyclohexyl oder Phenoxy.

Ganz besonders bevorzugt sind Verbindungen der Formel (I), bei welchen

$R^1$ für Isopropyl, Cyclopropyl oder s-Butyl steht und

$R^2$ für jeweils gegebenenfalls ein-bis dreifach durch Fluor und/oder Chlor substituiertes Methyl, Ethyl, n- oder i-Propyl, n-, i-, s- oder t-Butyl, Allyl, Propargyl, jeweils geradkettiges oder verzweigtes Pentyl, Hexyl, Heptyl, Octyl, Butenyl, Pentenyl, Hexenyl, Butinyl, Pentinyl oder Hexinyl steht; außerdem für jeweils gegebenenfalls ein- bis dreifach gleich oder verschieden durch Fluor, Chlor, Methyl, Ethyl und/oder Cyano substituiertes Cyclopropyl, Cyclopentyl, Cyclohexyl, Cyclohexenyl, Cyclopropylmethyl, Cyclopropylethyl, Cyclohexylmethyl, Cyclohexylethyl oder Cycloheptyl steht und schließlich für Benzyl oder Phenylethyl steht.

Im einzelnen seien die folgenden substituierten Triazolinone der allgemeinen Formel (I) genannt:

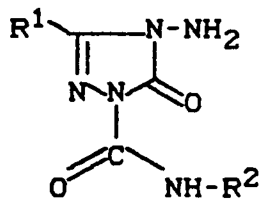

(I)

| $R^1$ | $R^2$ |
|---|---|
| $(CH_3)_2CH-$ | 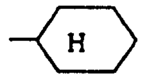 |
| $(CH_3)_2CH-$ | 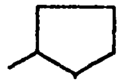 |
| $(CH_3)_2CH-$ | 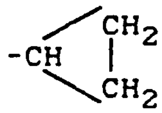 |
| $(CH_3)_2CH-$ | 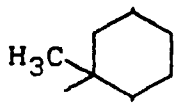 |
| $(CH_3)_2CH-$ | 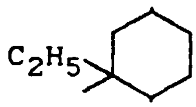 |
| $(CH_3)_2CH-$ | 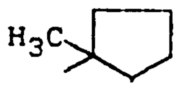 |
| $(CH_3)_2CH-$ | 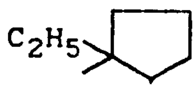 |
| $(CH_3)_2CH-$ | 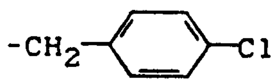 |
| $(CH_3)_2CH-$ | 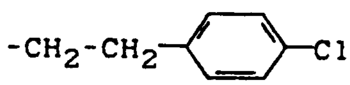 |
| $(CH_3)_2CH-$ | 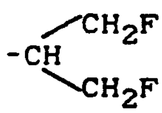 |
| $(CH_3)_2CH-$ | 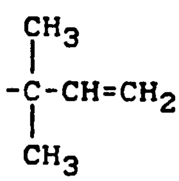 |

| $R^1$ | $R^2$ |
|---|---|
| $(CH_3)_2CH-$ | 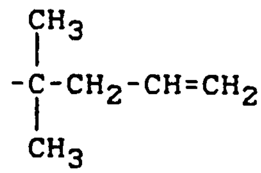 |
| $(CH_3)_2CH-$ | 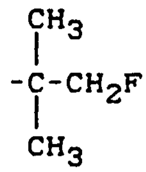 |
| $(CH_3)_2CH-$ | 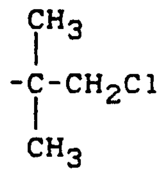 |
| $(CH_3)_2CH-$ | 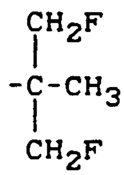 |
| $(CH_3)_2CH-$ | 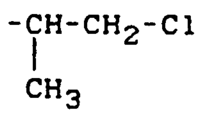 |
| $(CH_3)_2CH-$ | 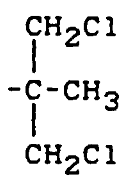 |
| $(CH_3)_2CH-$ | 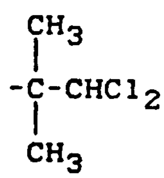 |
| $(CH_3)_2CH-$ | 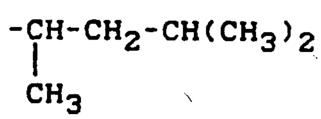 |

| $R^1$ | $R^2$ |
|---|---|
| $(CH_3)_2CH-$ | 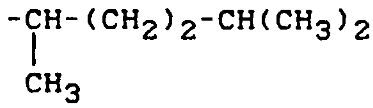 |
| $(CH_3)_2CH-$ | 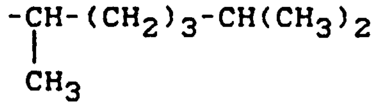 |
| $(CH_3)_2CH-$ | 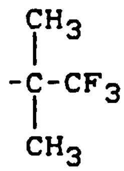 |
| $(CH_3)_2CH-$ | $-(CH_2)_2-CH_3$ |
| $(CH_3)_2CH-$ | $-(CH_2)_3-CH_3$ |
| $(CH_3)_2CH-$ | $-CH(CH_3)_2$ |
| $(CH_3)_2CH-$ | 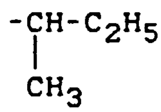 |
| $(CH_3)_2CH-$ | 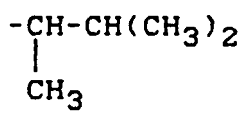 |
| $(CH_3)_2CH-$ | 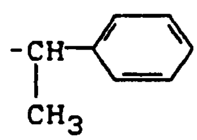 |
| $(CH_3)_2CH-$ | 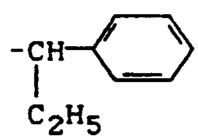 |
| $(CH_3)_2CH-$ | 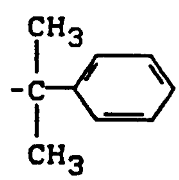 |

| $R^1$ | $R^2$ |
|---|---|
| $(CH_3)_2CH-$ | 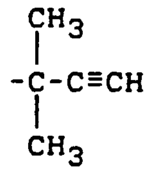 |
| $(CH_3)_2CH-$ | 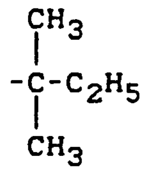 |
| $(CH_3)_2CH-$ | 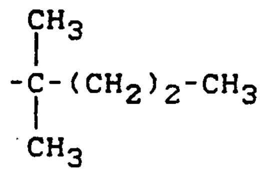 |
| $(CH_3)_2CH-$ | 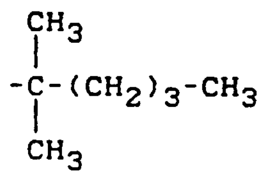 |
| $(CH_3)_2CH-$ | $-C(CH_3)_3$ |
| 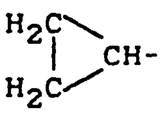 | 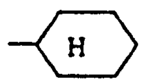 |
| 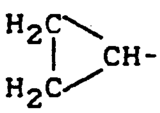 | 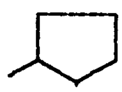 |
| 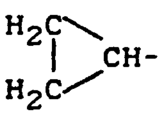 | 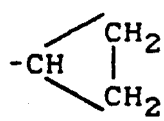 |
| 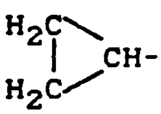 | 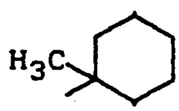 |
| 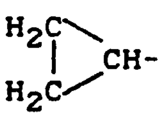 | 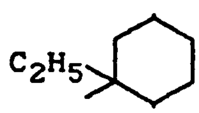 |

| $R^1$ | $R^2$ |
|---|---|
| 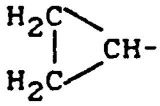 | 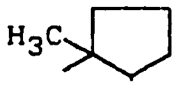 |
| 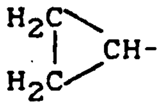 | 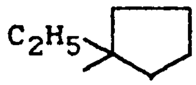 |
| 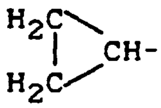 | 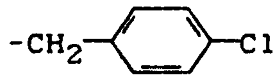 |
| 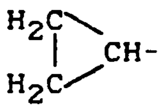 | 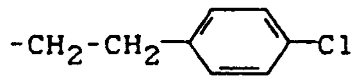 |
| 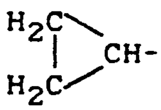 | 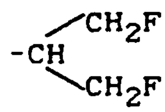 |
| 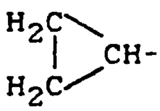 | 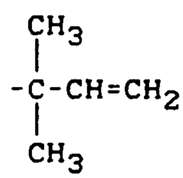 |
| 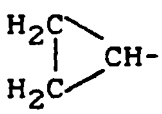 | 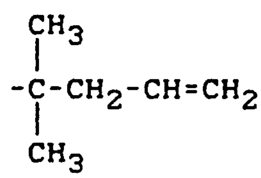 |
| 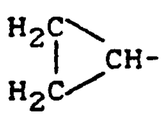 | 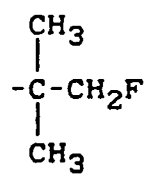 |
| 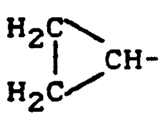 | 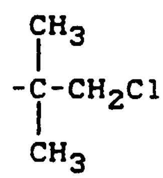 |

| $R^1$ | $R^2$ |
|---|---|
| 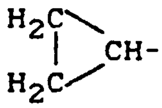 | 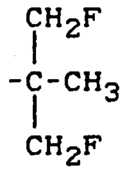 |
| 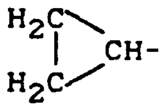 | 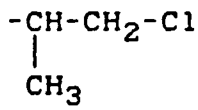 |
| 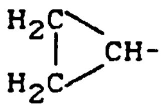 | 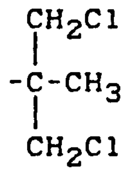 |
| 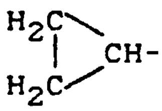 | 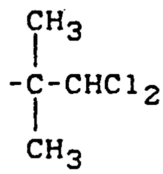 |
| 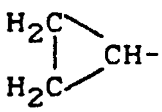 | 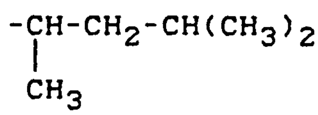 |
| 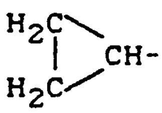 | 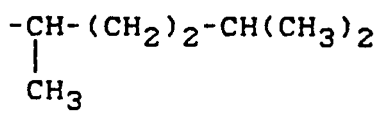 |
| 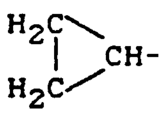 | 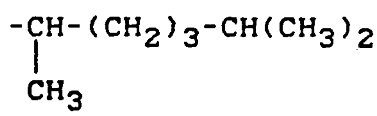 |
| 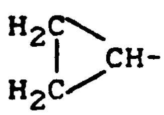 | 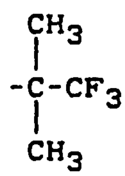 |

| $R^1$ | $R^2$ |
|---|---|
| 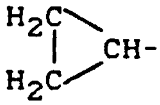 | $-(CH_2)_2-CH_3$ |
| 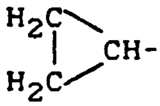 | $-(CH_2)_3-CH_3$ |
| 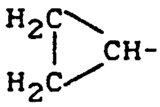 | $-CH(CH_3)_2$ |
| 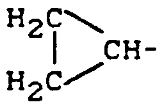 | 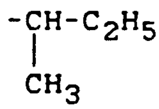 |
| 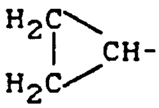 | 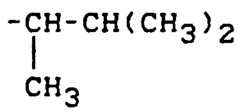 |
| 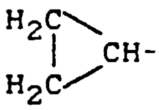 | 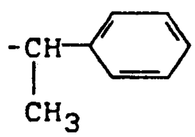 |
| 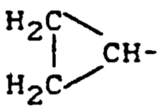 | 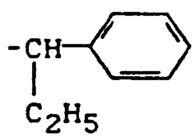 |
| 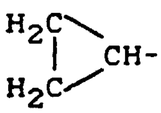 | 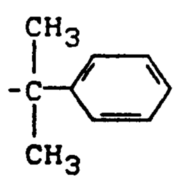 |
| 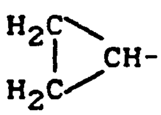 | 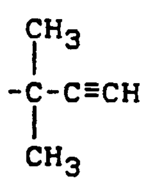 |

| $R^1$ | $R^2$ |
|---|---|
| 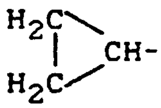 | 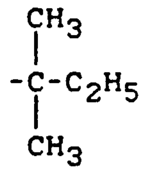 |
| 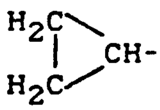 | 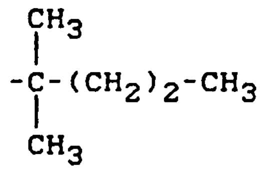 |
| 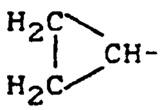 | 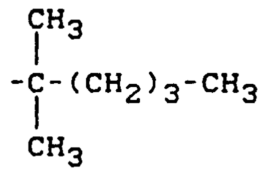 |
| 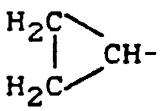 | $-C(CH_3)_3$ |
| 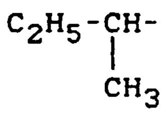 | 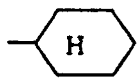 |
| 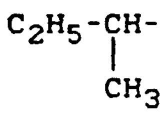 | 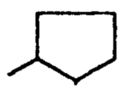 |
| 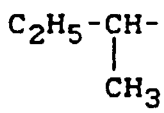 | 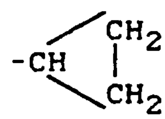 |
| 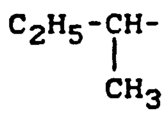 | 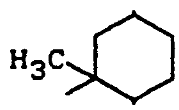 |

| $R^1$ | $R^2$ |
|---|---|
| 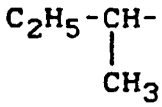 | 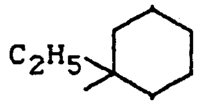 |
| 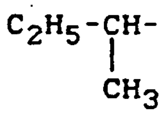 | 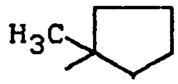 |
| 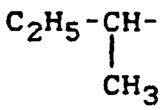 | 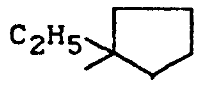 |
| 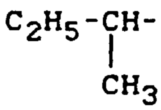 | 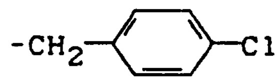 |
| 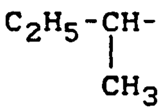 | 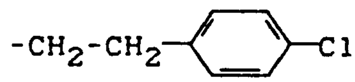 |
| 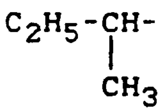 | 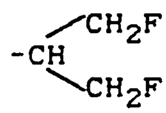 |
| 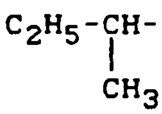 | 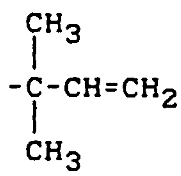 |
| 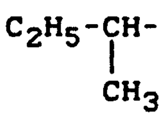 | 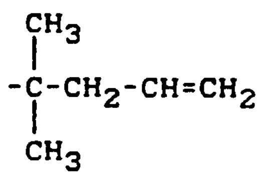 |

| $R^1$ | $R^2$ |
|---|---|
| 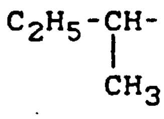 | 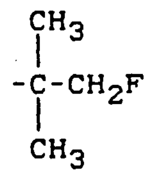 |
| 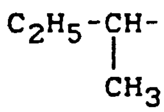 | 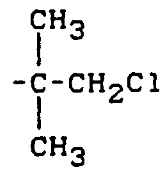 |
| 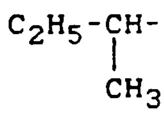 | 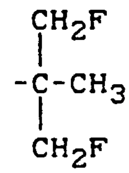 |
| 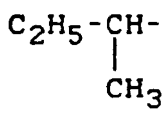 | 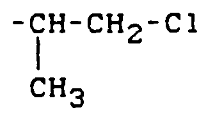 |
| 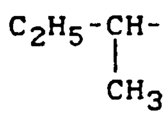 | 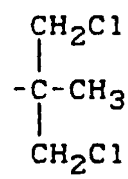 |
| 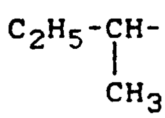 | 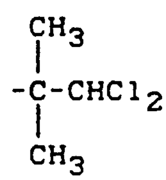 |
| 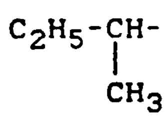 | 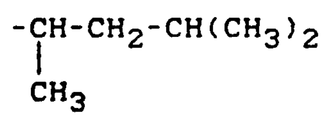 |
| 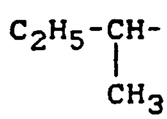 | 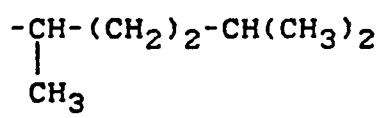 |
| 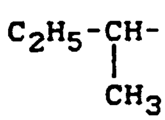 | 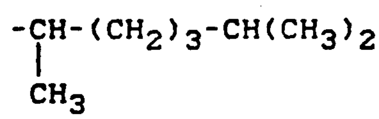 |

| $R^1$ | $R^2$ |
|---|---|
| 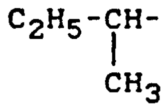 | 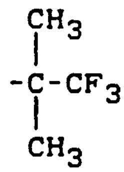 |
| 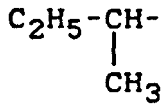 | $-(CH_2)_2-CH_3$ |
| 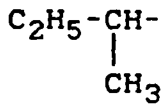 | $-(CH_2)_3-CH_3$ |
| 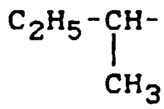 | $-CH(CH_3)_2$ |
| 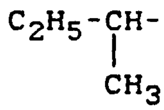 | 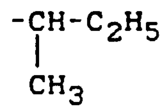 |
| 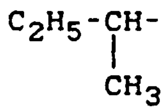 | 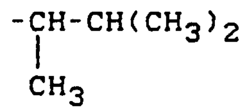 |
| 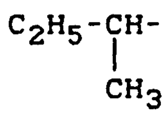 | 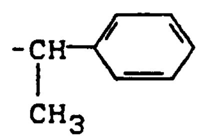 |
| 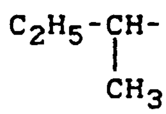 | 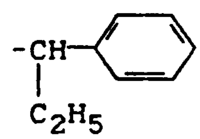 |
| 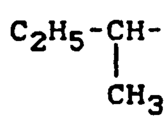 | 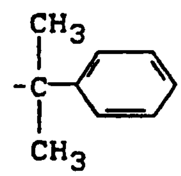 |

| $R^1$ | $R^2$ |
|---|---|
| 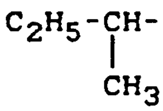 | 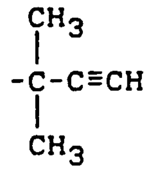 |
| 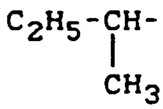 | 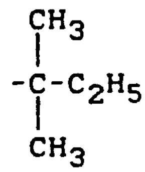 |
| 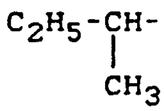 | 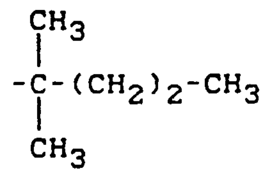 |
| 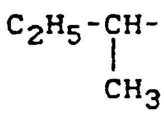 | 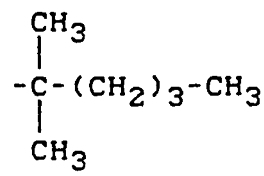 |
| 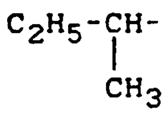 | $-C(CH_3)_3$ |

Verwendet man beispielsweise 1-(N-Isobutylcarbamoyl)-4-isopropylidenimino-3-isopropyl-1,2,4-triazolin-5-on als Ausgangsverbindung, so läßt sich der Reaktionsablauf des erfindungsgemäßen Verfahrens (a) durch das folgende Formelschema darstellen:

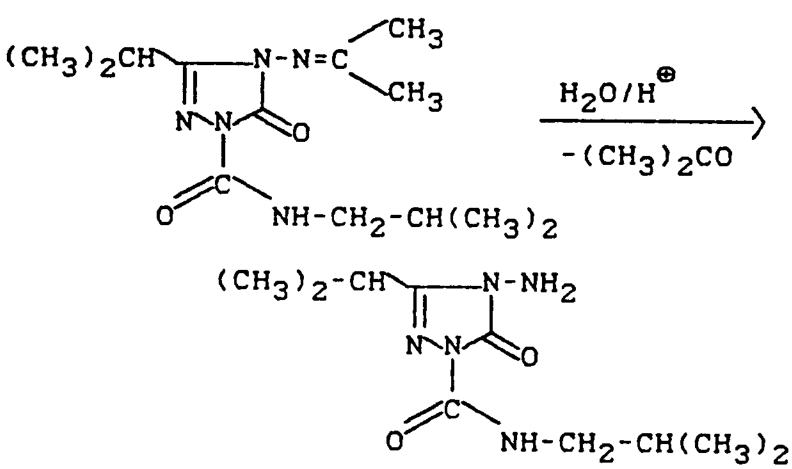


Verwendet man beispielsweise 4-Amino-3-cyclopropyl-1,2,4-(1H)-triazolin-5-on und t-Butylisocyanat als Ausgangsstoffe, so läßt sich der Reaktionsablauf des erfindungsgemäßen Verfahrens (b) durch das folgende Formelschema darstellen:

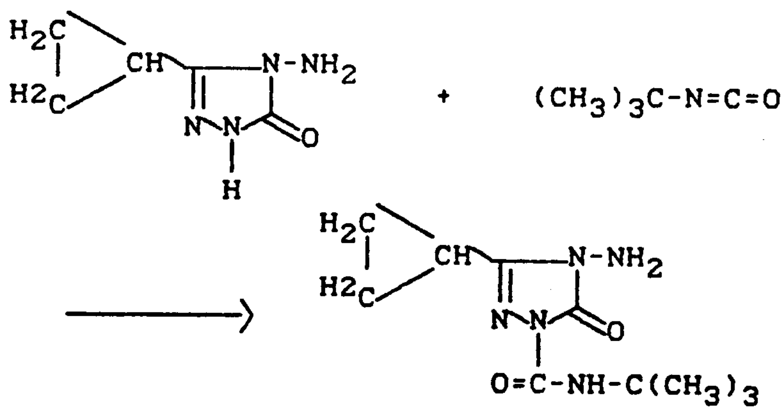

Verwendet man beispielsweise 1-Phenoxycarbonyl-4-amino-3-s-butyl-1,2,4-triazolin-5-on und N,N-Diethylpropan-1,3-diamin als Ausgangsstoffe, so läßt sich der Reaktionsablauf des erfindungsgemäßen Verfahrens (c) durch das folgende Formelschema darstellen:

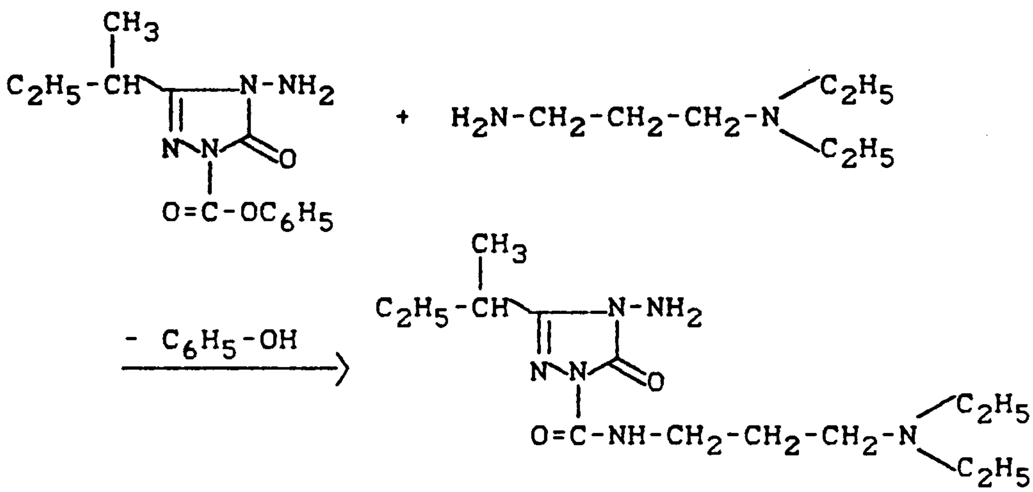


Verwendet man beispielsweise 4-Amino-3-isopropyl-1,2,4-(1H)-triazolin-5-on und N-Isopropyl-phenylcarbamat als Ausgangsstoffe, so läßt sich der Reaktionsablauf des erfindungsgemäßen Verfahrens (d) durch das folgende Formelschema darstellen:

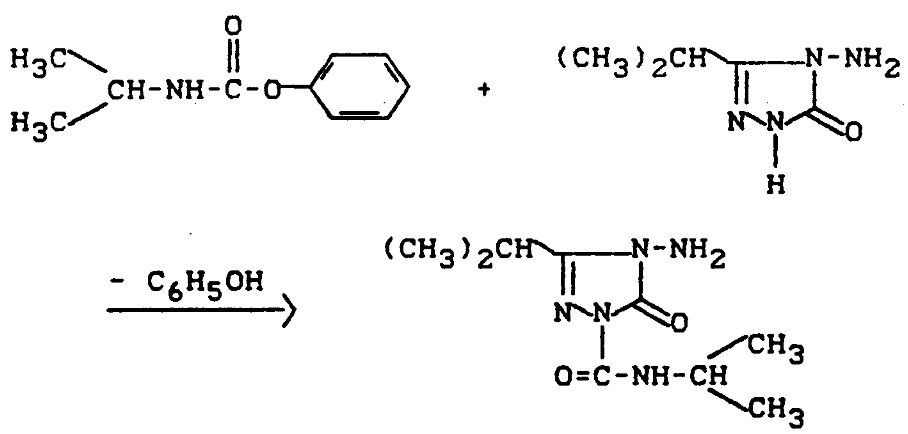


Die zur Durchführung des erfindungsgemäßen Verfahrens (a) als Ausgangsstoffe benötigten Hydrazone sind durch die Formel (II) allgemein definiert. In dieser Formel (II) stehen $R^1$ und $R^2$, vorzugsweise für diejenigen Reste, die bereits im Zusammenhang mit der Beschreibung der erfindungsgemäßen Stoffe der

Formel (I) als bevorzugt für diese Substituenten genannt wurden. $R^3$ und $R^4$ stehen vorzugsweise jeweils unabhängig voneinander für Wasserstoff, für geradkettiges oder verzweigtes Alkyl mit 1 bis 4 Kohlenstoffatomen oder für Phenyl oder Benzyl.

Die Hydrazone der Formel (II) sind noch nicht bekannt. Sie sind ebenfalls Gegenstand der vorliegenden Erfindung. Man erhält sie jedoch in Analogie zu bekannten Verfahren (vgl. z. B. Acta Pol. Pharm. 38, 153 - 162 [1981] bzw. C.A. 95: 203841j), beispielsweise wenn man 1-unsubstituierte 4-Amino-triazolinone der Formel (III),

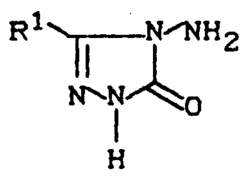

(III)

in welcher

$R^1$ die oben angegebene Bedeutung hat,

mit Aldehyden oder Ketonen der Formel (VIII),

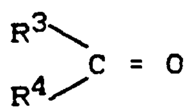

(VIII)

in welcher

$R^3$ und $R^4$ die oben angegebene Bedeutung haben,

gegebenenfalls in Gegenwart eines Verdünnungsmittels wie beispielsweise Dichlormethan oder Toluol und gegebenenfalls in Gegenwart eines Katalysators wie beispielsweise p-Toluolsulfonsäure bei Temperaturen zwischen 40 °C und 120 °C umsetzt und die so erhältlichen 1-unsubstituierten Triazolinon-Hydrazone der Formel (IX),

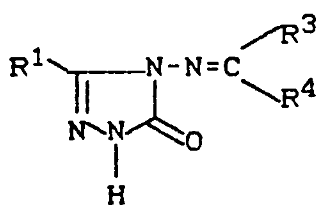

(IX)

in welcher

$R^1$, $R^3$ und $R^4$ die oben angegebene Bedeutung haben,

entweder in einer anschließenden 2. Stufe mit Isocyanaten der Formel (IV),

$R^2 - N = C = O$ (IV)

in welcher

$R^2$ die oben angegebene Bedeutung hat,

gegebenenfalls in Gegenwart eines Verdünnungsmittels wie beispielsweise Dichlormethan oder Dioxan und gegebenenfalls in Gegenwart eines Reaktionshilfsmittels wie beispielsweise Triethylamin bei Temperaturen zwischen 50 °C und 150 °C umsetzt;

oder alternativ in einer anschließenden 2. Stufe mit Chlorameisensäureestern der Formel (X),

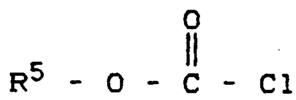 (X)

in welcher

$R^5$ für Alkyl, Aryl oder Arylalkyl steht,

gegebenenfalls in Gegenwart eines Verdünnungsmittels wie beispielsweise Tetrahydrofuran und gegebenenfalls in Gegenwart eines Reaktionshilfsmittels wie beispielsweise Natriumhydrid oder Kalium-t-butylat bei Temperaturen zwischen −20 °C und +40 °C umsetzt und die so erhältlichen Triazolinone der Formel (XI),

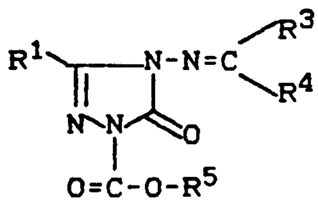 (XI)

in welcher

$R^1$, $R^3$, $R^4$ und $R^5$ die oben angegebene Bedeutung haben,

in einer anschließenden 3. Stufe mit Aminen der Formel (VI),

$R^2 - NH_2$ (VI)

in welcher

$R^2$ die oben angegebene Bedeutung hat,

gegebenenfalls in Gegenwart eines Verdünnungsmittels wie beispielsweise Tetrahydrofuran sowie gegebenenfalls in Gegenwart einer Base wie beispielsweise Natriumhydroxid, Kaliumhydroxid oder Diazabicycloundecen (DBU) bei Temperaturen zwischen 20 °C und 50 °C umsetzt.

Dabei ist es auch möglich und gegebenenfalls von Vorteil die Umsetzung der 1-unsubstituierten Triazolinon-Hydrazone der Formel (IX) mit Chlorameisensäureestern der Formel (X) und die anschließende Umsetzung der so erhältlichen Triazolinone (XI) mit Aminen der Formel (VI) in einem Reaktionsschritt in einem sogenannten Eintopfverfahren durchzuführen.

Die 1-unsubstituierten 4-Amino-triazolinone der Formel (III) sind teilweise bekannt und erhältlich in Analogie zu bekannten Verfahren (vergl. z.B. J.Heterocycl. Chem. 16, 403 [1979]; J. Heterocycl. Chem. 17, 1691 [1980]; Europ, J. Med. Chem. 18, 215 [1983]; Chem. Ber. 98, 3025 [1965]; Liebigs Ann. Chem. 637, 135 [1960]; J. Heterocycl. Chem. 21, 1769 − 1774 [1984]; Chim. Acta Turc. 7, 269 − 290 [1979] oder CA 106 (17): 138338e [1986]).

Die Aldehyde oder Ketone der Formel (VIII) sind allgemein bekannte Verbindungen der organischen Chemie.

Die 1-unsubstituierten Triazolinon-Hydrazone der Formel (IX) sind teilweise bekannt (vgl. z. B. Chim. Acta. Turc. 7, 269 − 290 [1979]).

Die Chlorameisensäureester der Formel (X) sind allgemein bekannte Verbindungen der organischen Chemie.

Die als Zwischenprodukte genannten Triazolinone der Formel (XI) sind noch nicht bekannt und ebenfalls Gegenstand der Erfindung.

$R^1$ steht dabei vorzugsweise für diejenigen Reste, die bereits im Zusammenhang mit der Beschreibung der erfindungsgemäßen Stoffe der Formel (I) als bevorzugt für diesen Substituenten genannt wurden.

$R^3$ und $R^4$ stehen vorzugsweise jeweils unabhängig voneinander für Wasserstoff, für geradkettiges oder verzweigtes Alkyl mit 1 bis 4 Kohlenstoffatomen oder für Phenyl oder Benzyl, und

$R^5$ steht vorzugsweise für geradkettiges oder verzweigtes Alkyl mit 1 bis 4 Kohlenstoffatomen oder für gegebenenfalls ein- bis dreifach, gleich oder verschieden substituiertes Phenyl oder Benzyl, wobei als Substituenten jeweils in Frage kommen: Halogen, Cyano, Nitro, jeweils geradkettiges oder verzweigtes Alkyl, Alkoxy oder Alkylthio mit jeweils 1 bis 4

Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^5$ steht insbesondere für Methyl, Ethyl, n- oder i-Propyl, n-, i-, s- oder t-Butyl oder für gegebenenfalls ein- bis zweifach, gleich oder verschieden substituiertes Phenyl oder Benzyl, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio.

Die zur Durchführung der erfindungsgemäßen Verfahren (b) und (d) als Ausgangsstoffe benötigten 1H-Triazolinone sind durch die Formel (III) allgemein definiert. In dieser Formel (III) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

Die 1H-Triazolinone der Formel (III) sind entweder bekannt oder erhältlich in Analogie zu bekannten Verfahren (vgl. z. B. J. Heterocycl. Chem. 16, 403 [1979]; J. Heterocycl. Chem. 17, 1691 [1980]; Europ. J. med. Chem. 18, 215 [1983]; Chem. Ber. 98, 3025 [1965]; Liebigs Ann. Chem. 637, 135 [1960]; J. Heterocycl. Chem. 21, 1769 - 1774 [1984]; Chim. Acta Turc. 7, 269 - 270 [1979]; CA 106 (17): 138338e [1986]).

Noch nicht bekannt und ebenfalls Gegenstand der Erfindung sind 1-unsubstituierte 4-Aminotriazolinone der Formel (IIIa),

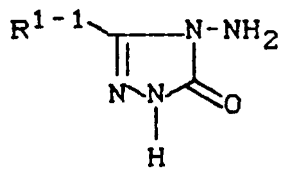

(IIIa)

in welcher

$R^{1-1}$ für n-Butyl, i-Butyl, s-Butyl oder Cyclopropyl, insbesondere für s-Butyl oder Cyclopropyl steht.

Man erhält sie in Analogie zu bekannten Verfahren (vgl. z. B. J. Heterocycl. Chem. 21, 1769 [1984] sowie Chem. Ber. 98, 3025 [1965]), beispielsweise, wenn man Hydrazinhydrat mit Diphenylcarbonat in üblicher Art und Weise umsetzt und das so erhältliche Kohlensäurehydrazid mit Carbonsäure-Derivaten der Formel (XII),

$R^{1-1} - COOR^6$ (XII)

in welcher

$R^{1-1}$ die oben angegebene Bedeutung hat, und

$R^6$ für Wasserstoff oder für geradkettiges oder verzweigtes Alkyl mit 1 bis 4 Kohlenstoffatomen steht,

ebenfalls in üblicher Art und Weise bei Temperaturen zwischen 150 °C und 250 °C cyclisiert (vgl. auch die Herstellungsbeispiele).

Carbonsäure-Derivate der Formel (XII) sind allgemein bekannte Verbindungen der organischen Chemie.

Die zur Durchführung des erfindungsgemäßen Verfahrens (b) weiterhin als Ausgangsstoffe benötigten Isocyanate sind durch die Formel (IV) allgemein definiert. In dieser Formel (IV) steht $R^2$ vorzugsweise für diejenigen Reste, die bereits im Zusammenhang mit der Beschreibung der erfindungsgemäßen Stoffe der Formel (I) als bevorzugt für diesen Substituenten genannt wurden.

Die Isocyanate der Formel (IV) sind allgemein bekannte Verbindungen der organischen Chemie. Die Verbindungen 2,2,2-Trifluorisopropylcyanat und 2,2,2-Trifluor-1,1-dimethylethylcyanat sind noch nicht bekannt, lassen sich jedoch nach bekannten Methoden herstellen.

Die zur Durchführung des erfindungsgemäßen Verfahrens (c) als Ausgangsstoffe benötigten Triazolinone sind durch die Formel (V) allgemein definiert. In dieser Formel (V) steht $R^1$ vorzugsweise für diejenigen Reste, die bereits im Zusammenhang mit der Beschreibung der erfindungsgemäßen Stoffe der Formel (I) als bevorzugt für diesen Substituenten genannt wurden.

$R^5$ steht vorzugsweise für geradkettiges oder verzweigtes Alkyl mit 1 bis 4 Kohlenstoffatomen oder für gegebenenfalls ein- bis dreifach, gleich oder verschieden substituiertes Phenyl oder Benzyl,

wobei als Substituenten jeweils infrage kommen: Halogen, Cyano, Nitro, jeweils geradkettiges oder verzweigtes Alkyl, Alkoxy oder Alkylthio mit jeweils 1 bis 4 Kohlenstoffatomen oder jeweils geradkettiges oder verzweigtes Halogenalkyl, Halogenalkoxy oder Halogenalkylthio mit jeweils 1 bis 4 Kohlenstoffatomen und 1 bis 9 gleichen oder verschiedenen Halogenatomen;

$R^5$ steht insbesondere für Methyl, Ethyl, n- oder i-Propyl, n-, i-, s- oder t-Butyl oder für gegebenenfalls ein- bis zweifach, gleich oder verschieden substituiertes Phenyl oder Benzyl, wobei als Substituenten jeweils in Frage kommen: Fluor, Chlor, Brom, Cyano, Nitro, Methyl, Ethyl, n- oder i-Propyl, n-, i-, s- oder t-Butyl, Methoxy, Ethoxy, n- oder i-Propoxy, Methylthio, Trifluormethyl, Trifluormethoxy oder Trifluormethylthio.

Die Triazolinone der Formel (V) sind noch nicht bekannt.

Man erhält sie in Analogie zu bekannten Verfahren, wenn man 1H-Triazolinone der Formel (III),

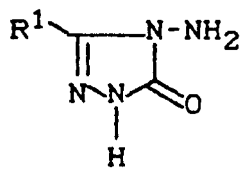

(III)

in welcher

$R^1$ die oben angegebene Bedeutung hat,

mit Chlorameisensäureestern der Formel (X),

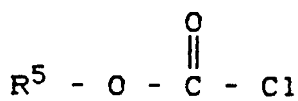

(X)

in welcher

$R^5$ die oben angegebene Bedeutung hat,

gegebenenfalls in Gegenwart eines Verdünnungsmittels wie beispielsweise Tetrahydrofuran und gegebenenfalls in Gegenwart eines Reaktionshilfsmittels wie beispielsweise Kalium-t-butylat oder Natriumhydrid bei Temperaturen zwischen -20 °C und +40 °C umsetzt (vgl. auch die Herstellungsbeispiele).

Die zur Durchführung des erfindungsgemäßen Verfahrens (d) weiterhin als Ausgangsstoffe benötigten Urethane sind durch die Formel (VII) allgemein definiert. In dieser Formel (VII) steht $R^2$ vorzugsweise für diejenigen Reste, die bereits im Zusammenhang mit der Beschreibung der erfindungsgemäßen Stoffe der Formel (I) als bevorzugt für diese Substituenten genannt wurden. $R^5$ steht vorzugsweise für diejenigen Reste, die bereits im Zusammenhang mit der Beschreibung der Vorprodukte der Formel (V) als bevorzugt oder besonders bevorzugt für diesen Substituenten genannt wurden.

Die Urethane der Formel (VII) sind allgemein bekannte Verbindungen der organischen Chemie oder erhältlich mit Hilfe allgemein bekannter Verfahren.

Als Säuren zur Durchführung des erfindungsgemäßen Verfahrens (a) kommen alle üblicherweise für Hydrazonspaltungen verwendbaren anorganischen und organischen Säuren in Frage. Vorzugsweise verwendet man anorganische Mineralsäuren wie Chlorwasserstoffsäure, Schwefelsäure oder Phosphorsäure.

Als Verdünnungsmittel zur Durchführung des erfindungsgemäßen Verfahrens (a) kommen alle üblichen organischen oder anorganischen Lösungsmittel in Frage. Vorzugsweise verwendet man polare mit Wasser mischbare organische Lösungsmittel, insbesondere Alkohole, wie Methanol, Ethanol, Propanol oder Butanol, deren Gemische mit Wasser oder reines Wasser als Verdünnungsmittel.

Die Reaktionstemperaturen können bei der Durchführung des erfindungsgemäßen Verfahrens (a) in einem größeren Bereich variiert werden. Im allgemeinen arbeitet man bei Temperaturen zwischen 20 °C und 150 °C, vorzugsweise bei Temperaturen zwischen 50 °C und 120 °C.

Das erfindungsgemäße Verfahren (a) wird üblicherweise bei Normaldruck oder unter vermindertem Druck durchgeführt. Arbeitet man unter vermindertem Druck, so kommen Druckbereiche zwischen 20 und 400 mbar, vorzugsweise zwischen 100 und 200 mbar in Frage.

Zur Durchführung des erfindungsgemäßen Verfahrens (a) setzt man pro Mol an Hydrazon der Formel (II) im allgemeinen 1 bis 50 Mol, vorzugsweise 1 bis 20 Mol an Säure ein. Dabei löst man das Hydrazon der

Formel (II) in einer geeigneten Menge an Verdünnungsmittel, setzt dann die erforderliche Menge Säure zu und engt die Mischung unter vermindertem Druck über mehrere Stunden langsam ein.

In einer besonderen Durchführungsform ist es auch möglich, das erfindungsgemäße Verfahren (a) und die Herstellung der dafür erforderlichen Vorprodukte der Formel (II) in einem Reaktionsschritt in einem sogenannten Eintopfverfahren durchzuführen.

Dabei gibt es die Möglichkeit, als Ausgangsverbindungen die Triazolinone der Formel (XI) zu wählen und diese nacheinander im Eintopfverfahren mit Aminen der Formel (VI) und anschließend mit Säure gemäß dem erfindungsgemäßen Verfahren (a) umzusetzen (vgl. hierzu auch die Herstellungsbeispiele) oder alternativ, als Ausgangsverbindungen die Triazolin-Hydrazone der Formel (IX) zu wählen und diese nacheinander im Eintopfverfahren mit Chlorameisensäureestern der Formel (X), dann mit Aminen der Formel (VI) und anschließend mit Säure gemäß dem erfindungsgemäßen Verfahren (a) umzusetzen.

Als Verdünnungsmittel zur Durchführung des erfindungsgemäßen Verfahrens (b) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester.

Das erfindungsgemäße Verfahren (b) wird gegebenenfalls in Gegenwart eines geeigneten Reaktionshilfsmittels durchgeführt. Als solche kommen alle üblichen anorganischen oder organischen Basen in Frage. Hierzu gehören beispielsweise tertiäre Amine, wie Triethylamin, N,N-Dimethylanilin, N,N-Diethylbenzylamin, N,N-Dimethylcyclohexylamin oder Dibutylzinndilaureat, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung des erfindungsgemäßen Verfahrens (b) in einem größeren Bereich variiert werden. Im allgemeinen arbeitet man bei Temperaturen zwischen 0 °C und 150 °C, vorzugsweise bei Temperaturen zwischen 20 °C und 100 °C.

Zur Durchführung des erfindungsgemäßen Verfahrens (b) setzt man pro Mol an 1H-Triazolinon der Formel (III) im allgemeinen 1.0 bis 2.0 Mol, vorzugsweise 1.0 bis 1.5 Mol an Isocyanat der Formel (IV) und gegebenenfalls 0.001 bis 2.0 Mol, vorzugsweise 0.001 bis 1.0 Mol an Reaktionshilfsmittel ein.
Die Reaktionsdurchführung, Aufarbeitung und Isolierung der Reaktionsprodukte erfolgt nach allgemein üblichen Methoden.

Als Verdünnungsmittel zur Durchführung der erfindungsgemäßen Verfahren (c) und (d) kommen inerte organische Lösungsmittel in Frage. Hierzu gehören insbesondere aliphatische, alicyclische oder aromatische, gegebenenfalls halogenierte Kohlenwasserstoffe, wie beispielsweise Benzin, Benzol, Toluol, Xylol, Chlorbenzol, Petrolether, Hexan, Cyclohexan, Dichlormethan, Chloroform, Tetrachlorkohlenstoff, Ether, wie Diethylether, Dioxan, Tetrahydrofuran oder Ethylenglykoldimethyl- oder -diethylether, Nitrile, wie Acetonitril oder Propionitril, Amide, wie Dimethylformamid, Dimethylacetamid, N-Methylformanilid, N-Methylpyrrolidon oder Hexamethylphosphorsäuretriamid oder Ester, wie Essigsäureethylester, oder Sulfoxide, wie Dimethylsulfoxid.

Die erfindungsgemäßen Verfahren (c) und (d) können gegebenenfalls in Gegenwart eines geeigneten Reaktionshilfsmittels durchgeführt werden. Als solche kommen alle üblichen anorganischen oder organischen Basen in Frage. Hierzu gehören beispielsweise Alkalimetallhydroxide, wie Natriumhydroxid oder Kaliumhydroxid, Alkalimetallcarbonate, wie Natriumcarbonat, Kaliumcarbonat oder Natriumhydrogencarbonat, sowie tertiäre Amine, wie Triethylamin, N,N-Dimethylanilin, Pyridin, N,N-Dimethylaminopyridin, Diazabicyclooctan (DABCO), Diazabicyclononen (DBN) oder Diazabicycloundecen (DBU).

Die Reaktionstemperaturen können bei der Durchführung der erfindungsgemäßen Verfahren (c) und (d) in einem größeren Bereich variiert werden. Im allgemeinen arbeitet man bei Temperaturen zwischen 0 °C und 120 °C, vorzugsweise bei Temperaturen zwischen 20 °C und 50 °C.

Zur Durchführung des erfindungsgemäßen Verfahrens (c) setzt man pro Mol an Triazolinon der Formel (V) im allgemeinen 1.0 bis 5.0 Mol, vorzugsweise 1.0 bis 2.5 Mol an Amin der Formel (VI) und gegebenenfalls 1.0 bis 2.0 Mol, vorzugsweise 1.0 bis 1.2 Mol an Reaktionshilfsmittel ein.
Die Reaktionsdurchführung, Aufarbeitung und Isolierung der Reaktionsprodukte erfolgt nach allgemein üblichen Methoden.

In einer besonderen Durchführungsform ist es auch möglich, das erfindungsgemäße Verfahren (c) und die Herstellung der dafür erforderlichen Vorprodukte der Formel (V) in einem Reaktionsschritt in einem sogenannten Eintopfverfahren durchzuführen.

Man geht dabei aus von 1H-Triazolinonen der Formel (III) und setzt diese nacheinander im Eintopfverfahren zunächst mit Chlorameisensäureestern der Formel (X) und anschließend mit Aminen der Formel

(VI) gemäß dem erfindungsgemäßen Verfahren (c) um.

Zur Durchführung des erfindungsgemäßen Verfahrens (d) setzt man pro Mol an 1H-Triazolinon der Formel (III) im allgemeinen 1.0 bis 5.0 Mol, vorzugsweise 1.0 bis 2.5 Mol an Urethan der Formel (VII) und gegebenenfalls 1.0 bis 5.0 Mol, vorzugsweise 1.0 bis 2.5 Mol an Reaktionshilfmittel ein.
Die Reaktionsdurchführung, Aufarbeitung und Isolierung der Reaktionsprodukte erfolgt nach allgemein üblichen Methoden.

Eine weitere Methode, erfindungsgemäße Verbindungen der Formel (I) zu erhalten, besteht darin, daß man Oxadiazolinone der Formel (XIII),

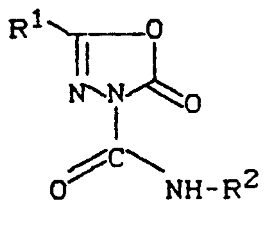

(XIII)

in welcher

$R^1$ und $R^2$ die oben angegebene Bedeutung haben,

mit Hydrazinhydrat in Gegenwart eines geeigneten Verdünnungsmittels wie beispielsweise Methanol oder Ethanol bei Temperaturen zwischen 20 °C und 100 °C umsetzt und die so erhältlichen Carbazidsäure-Derivate der Formel (XIV),

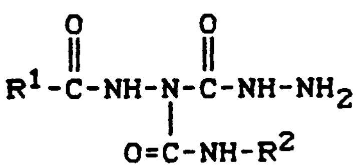

(XIV)

in welcher

$R^1$ und $R^2$ die oben angegebene Bedeutung haben,

in Gegenwart eines geeigneten Verdünnungsmittels wie beispielsweise Toluol, Chlorbenzol oder Dichlorbenzol bei Temperaturen zwischen 80 °C und 200 °C thermisch cyclisiert.

Oxadiazolinone der Formel (XIII) sind bekannt (vgl. z. B. FR 14 15 605 bzw. C.A. 64: P5105g sowie NL 6 510 645 bzw. C.A. 65: P2274d-f) oder erhältlich nach allgemein bekannten Verfahren beispielsweise durch Umsetzung der entsprechenden 4H-Oxadiazolinone mit Isocyanaten der Formel (IV) in Analogie zur Durchführung des erfindungsgemäßen Verfahrens (b) oder zur Synthese der Vorprodukte der Formel (II).

Die Reinigung der Endprodukte der Formel (I) erfolgt mit Hilfe üblicher Verfahren, beispielsweise säulenchromatographisch oder durch Umkristallisieren.
Die Charakterisierung erfolgt mit Hilfe des Schmelzpunktes oder bei nicht kristallisierenden Verbindungen mit Hilfe des Protonen-Kernresonanzspektrums.

Die erfindungsgemäßen Wirkstoffe können als Defoliants, Desiccants, Krautabtötungsmittel und insbesondere als Unkrautvernichtungsmittel verwendet werden. Unter Unkraut im weitesten Sinne sind alle Pflanzen zu verstehen, die an Orten aufwachsen, wo sie unerwünscht sind.

Ob die erfindungsgemäßen Stoffe als totale oder selektive Herbizide wirken, hängt im wesentlichen von der angewendeten Menge ab.

Die erfindungsgemaßen Wirkstoffe können z.B. bei den folgenden Pflanzen verwendet werden:

Dikotyle Unkräuter der Gattungen:

Sinapis, Lepidium, Galium, Stellaria, Matricaria, Anthemis, Galinsoga, Chenopodium, Urtica, Senecio, Amaranthus, Portulaca, Xanthium, Convolvulus, Ipomoea, Polygonum, Sesbania, Ambrosia, Cirsium, Carduus, Sonchus, Solanum, Rorippa, Rotala, Lindernia, Lamium, Veronica, Abutilon, Emex, Datura, Viola, Galeopsis, Papaver, Centaurea und Mercurialis.

Dikotyle Kulturen der Gattungen:

Gossypium, Glycine, Beta, Daucus, Phaseolus, Pisum, Solanum, Linum, Ipomoea, Vicia, Nicotiana, Lycopersicon, Arachis, Brassica, Lactuca, Cucumis, Cucurbita.

Monokotyle Unkräuter der Gattungen:

Echinochloa, Setaria, Panicum, Digitaria, Phleum, Poa, Festuca, Eleusine, Brachiaria, Lolium, Bromus, Avena, Cyperus, Sorghum, Agropyron, Cynodon, Monochoria, Fimbristylis, Sagittaria, Eleocharis, Scirpus, Paspalum, Ischaemum, Sphenoclea, Dactyloctenium, Agrostis, Alopecurus, Apera.

Monokotyle Kulturen der Gattungen:

Oryza, Zea, Triticum, Hordeum, Avena, Secale, Sorghum, Panicum, Saccharum, Ananas, Asparagus, Allium.

Die Verwendung der erfindungsgemäßen Wirkstoffe ist jedoch keineswegs auf diese Gattungen beschränkt, sondern erstreckt sich in gleicher Weise auch auf andere Pflanzen.

Die Verbindungen eignen sich in Abhängigkeit von der Konzentration zur Totalunkrautbekämpfung z.B. auf Industrie- und Gleisanlagen und auf Wegen und Plätzen mit und ohne Baumbewuchs. Ebenso können die Verbindungen zur Unkrautbekämpfung in Dauerkulturen, z.B. Forst, Ziergehölz-, Obst-, Wein-, Citrus-, Nuß-, Bananen-, Kaffee-, Tee-, Gummi-, Ölpalm-, Kakao-, Beerenfrucht- und Hopfenanlagen und zur selektiven Unkrautbekämpfung in einjährigen Kulturen eingesetzt werden.

Dabei lassen sich die erfindungsgemäßen Wirkstoffe mit besonders gutem Erfolg zur Bekämpfung mono- und dikotyler Unkräuter in mono- und dikotylen Kulturen wie beispielsweise Zuckerrüben, Mais, Weizen und Gerste einsetzen.

Die Wirkstoffe können in die üblichen Formulierungen überführt werden, wie Lösungen, Emulsionen, Spritzpulver, Suspensionen, Pulver, Stäubemittel, Pasten, lösliche Pulver, Granulate, Suspensions-Emulsions-Konzentrate, Wirkstoff-imprägnierte Natur und synthetische Stoffe sowie Feinstverkapselungen in polymeren Stoffen.

Diese Formulierungen werden in bekannter Weise hergestellt, z. B. durch Vermischen der Wirkstoffe mit Streckmitteln, also flüssigen Lösungsmitteln und/oder festen Trägerstoffen, gegebenenfalls unter Verwendung von oberflächenaktiven Mitteln, also Emulgiermitteln und/oder Dispergiermitteln und/oder schaumerzeugenden Mitteln.

Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten und chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, mineralische und pflanzliche Öle, Alkohole, wie Butanol oder Glykol sowie deren Ether und Ester, Ketone wie Aceton, Methylethylketon, Methylisobutylketon oder Cyclohexanon, stark polare Lösungsmittel, wie Dimethylformamid und Dimethylsulfoxid, sowie Wasser.

Als feste Trägerstoffe kommen in Frage:

Z.B. Ammoniumsalze und natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate, als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengeln; als Emulgier- und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylaryl-polyglykolether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0,1 und 95 Gewichtsprozent Wirkstoff, vorzugsweise zwischen 0,5 und 90 %.

Die erfindungsgemäßen Wirkstoffe können als solche oder in ihren Formulierungen auch in Mischung mit bekannten Herbiziden zur Unkrautbekämpfung Verwendung finden, wobei Fertigformulierungen oder Tankmischungen möglich sind.

Für die Mischungen kommen bekannte Herbizide wie z.B. 1-Amino-6-ethylthio-3-(2,2-dimethylpropyl)-1,3,5-triazin-2,4(1H,3H)-dion (AMETHYDIONE) oder N-(2-Benzthiazolyl)-N,N'-dimethylharnstoff (METABENZTHIAZURON) zur Unkrautbekämpfung in Getreide; 4-Amino-3-methyl-6-phenyl-1,2,4-triazin-5(4H)-on (METAMITRON) zur Unkrautbekämpfung in Zuckerrüben und 4-Amino-6-(1,1-dimethylethyl)-3-methylthio-1,2,4-triazin-5(4H)-on (METRIBUZIN) zur Unkrautbekämpfung in Sojabohnen, in Frage. Auch Mischungen mit 2,4-Dichlorphenoxyessigsäure (2,4-D); 4-(2,4-Dichlorphenoxy)-buttersäure (2,4-DB); 2,4-Dichlorphenoxypropionsäure (2,4-DP); Chloressigsäure-N-(methoxymethyl)-2,6-diethylanilid (ALACHLOR); 2-Chlor-4-ethylamino-6-isopropylamino-1,3,5-triazin (ATRAZIN); Methyl-5-(2,4-dichlorphenoxy)-2-nitrobenzoat (BIFENOX); 3,5-Dibrom-4-hydroxy-benzonitril (BROMOXYNIL); 2-Chlor-N-{[(4-methoxy-6-methyl-1,3,5-triazin-2-yl)-amino]-carbonyl}-benzolsulfonamid (CHLORSULFURON); N,N-Dimethyl-N'-(3-chlor-4-methylphenyl)-harnstoff (CHLORTOLURON); 2-Chlor-4-ethylamino-6-(3-cyanopropylamino)-1,3,5-triazin (CYANAZIN); 2-[4-(2,4-Dichlorphenoxy)-phenoxy]-propionsäure, deren Methyl- oder deren Ethylester (DICLOFOP); 3,6-Dichlor-2-pyridincarbonsäure (CLOPYRALID); N,N-Di-n-propyl-thiocarbamidsäure-S-ethylester (EPTAME); 4-Amino-6-t-butyl-3-ethylthio-1,2,4-triazin-5(4H)-on (ETHIOZIN); 2-{4-[(6-Chlor-2-benzoxazolyl)-oxy]-phenoxy}-propansäure, deren Methyl- oder deren Ethylester (FENOXAPROP); [(4-Amino-3,5-dichlor-6-fluor-2-pyridinyl)-oxy]-essigsäure bzw. deren 1-Methylheptylester (FLUROXYPYR); Methyl-2-[4,5-dihydro-4-methyl-4-(1-methylethyl)-5-oxo-1H-imidazol-2-yl]-4(5)-methylbenzoat (IMAZAMETHABENZ); 3,5-Diiod-4-hydroxybenzonitril (IOXYNIL); N,N-Dimethyl-N'-(4-isopropylphenyl)-harnstoff (ISOPROTURON); (2-Methyl-4-chlorphenoxy)-essigsäure (MCPA); (4-Chlor-2-methylphenoxy)-propionsäure (MCPP); N-Methyl-2-(1,3-benzthiazol-2-yloxy)-acetanilid (MEFENACET); 2-Ethyl-6-methyl-N-(1-methyl-2-methoxyethyl)-chloracetanilid (METOLACHLOR); 2-{[[((4-Methoxy-6-methyl-1,3,5-triazin-2-yl)-amino)-carbonyl]-amino]-sulfonyl}-benzoesäure oder deren Methylester (METSULFURON); (2-Chlor-4-trifluormethylphenyl)-(3-ethoxy-4-nitro-phenyl)-ether (OXYFLUORFEN); N-(1-Ethylpropyl)-3,4-dimethyl-2,6-dinitroanilin (PENDIMETHALIN); 2-Chlor-N-isopropylacetanilid (PROPACHLOR); 0-(6-Chlor-3-phenyl-pyridazin-4-yl)-S-octyl-thiocarbonat (PYRIDATE); 2-Chlor-4,6-bis-(ethylamino)-1,3,5-triazin (SIMAZIN); 4-Ethylamino-2-t-butylamino-6-methylthio-s-triazin (TERBUTRYNE); 3-[[[[(4-Methoxy-6-methyl-1,3,5-triazin-2-yl)-amino]-carbonyl]-amino]-sulfonyl]-thiophen-2-carbonsäure-methylester (THIAMETURON); N,N-Diisopropyl-S-(2,3,3-trichlorallyl)-thiolcarbamat (TRIALLATE) und 2,6-Dinitro-4-trifluormethyl-N,N-dipropylanilin (TRIFLURALIN) sind möglich. Einige Mischungen zeigen überraschenderweise auch synergistische Wirkung.

Auch eine Mischung mit anderen bekannten Wirkstoffen, wie Fungiziden, Insektiziden, Akariziden, Nematiziden, Schutzstoffen gegen Vogelfraß, Pflanzennährstoffen und Bodenstrukturverbesserungsmitteln ist möglich.

Die Wirkstoffe können als solche, in Form ihrer Formulierungen oder den daraus durch weiteres Verdünnen bereiteten Anwendungsformen, wie gebrauchsfertige Lösungen, Suspensionen, Emulsionen, Pulver, Pasten und Granulate angewandt werden. Die Anwendung geschieht in üblicher Weise, z.B. durch Gießen, Spritzen, Sprühen, Streuen.

Die erfindungsgemäßen Wirkstoffe können sowohl vor als auch nach dem Auflaufen der Pflanzen appliziert werden.

Sie können auch vor der Saat in den Boden eingearbeitet werden.

Die angewandte Wirkstoffmenge kann in einem größeren Bereich schwanken. Sie hängt im wesentlichen von der Art des gewünschten Effektes ab. Im allgemeinen liegen die Aufwandmengen zwischen 0,01 und 10 kg Wirkstoff pro Hektar Bodenfläche, vorzugsweise zwischen 0,05 und 5 kg pro ha.

Die Herstellung und die Verwendung der erfindungsgemäßen Wirkstoffe geht aus den nachfolgenden Beispielen hervor.

Herstellungsbeispiele

Beispiel 1

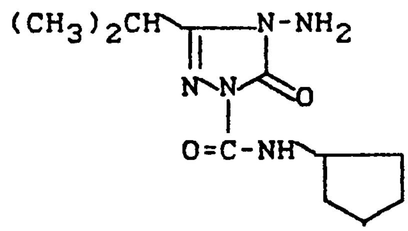

(Verfahren (b))

Zu 7,1 g (0,05 Mol) 4-Amino-3-isopropyl-(1H)-1,2,4-triazolin-5-on in 100 ml absolutem Acetonitril gibt man 6,1 g (0,055 Mol) Cyclopentylisocyanat und eine kleine Menge Diazabicycloundecen (DBU), rührt 12 Stunden bei Raumtemperatur, engt dann im Vakuum ein, nimmt den Rückstand in Dichlormethan auf, wäscht mit Wasser neutral trocknet über Magnesiumsulfat, engt ein und kristallisiert den Rückstand durch Verreiben mit Diethylether.

Man erhält 10,0 g (78 % der Theorie) an 4-Amino-1-(N-cyclopentylcarbamoyl)-3-isopropyl-1,2,4-triazolin-5-on vom Schmelzpunkt 109 °C.

Beispiel 2

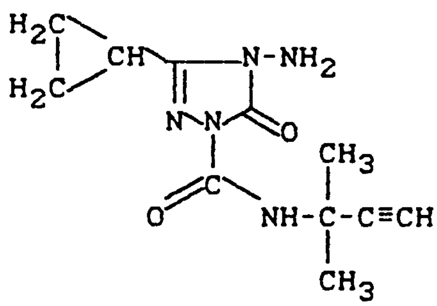

(Verfahren (a) – Eintopfvariante)

Zu 11,1 g (0,05 Mol) 4-(4-Methylpent-2-ylidenimino)-3-cyclopropyl-1,2,4-(1H)-triazolin-5-on in 150 ml absolutem Acetonitril gibt man 5,5 g (0,05 Mol) 3-Methyl-3-butinylisocyanat und eine kleine Menge an Diazabicycloundecen (DBU), rührt 12 Stunden bei 20 °C, engt anschließend im Vakuum ein, nimmt den Rückstand in wäßrigem Ethanol auf, gibt 2 ml konzentrierte Salzsäure zu, engt bei 60 °C langsam unter vermindertem Druck ein, nimmt den Rückstand in Dichlormethan auf, wäscht mit gesättigter Natriumhydrogencarbonatlösung, trocknet über Magnesiumsulfat, engt ein und kristallisiert den Rückstand durch Verreiben mit Diethylether.

Man erhält 10,4 g (84 % der Theorie) an 4-Amino-3-cyclopropyl-1-[N-(3-methyl-3-butinyl)-carbamoyl]-1,2,4-triazolin-5-on vom Schmelzpunkt 160 °C.

In entsprechender Weise und gemäß den allgemeinen Angaben zur Herstellung erhält man die folgenden substituierten Triazolinone der allgemeinen Formel (I):

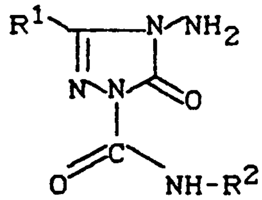

(I)

| Bsp.-Nr. | $R^1$ | $R^2$ | physikalische Konstante |
|---|---|---|---|
| 3 | $(CH_3)_3C-$ | $-(CH_2)_5-CH_3$ | $n_D^{20}$: 1,4980 |
| 4 | $CH_3-(CH_2)_2-$ | 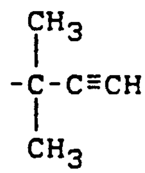 | Fp. 106 °C |
| 5 | $(CH_3)_2CH-$ | 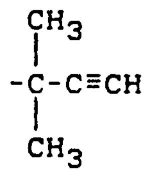 | Fp. 90 °C |
| 6 | $(CH_3)_2CH-$ | 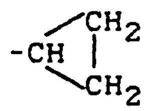 | Fp. 133 °C |
| 7 | $CH_3-(CH_2)_2-$ | 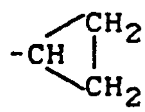 | Fp. 98 °C |
| 8 | $(CH_3)_3C-$ | 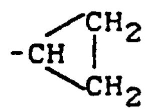 | Fp. 158 °C |
| 9 | $CH_3-(CH_2)_2-$ | 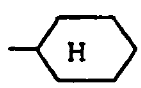 | Fp. 100 °C |
| 10 | $(CH_3)_2CH-$ | 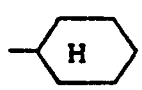 | $n_D^{20}$: 1,5168 |

| Bsp.-Nr. | $R^1$ | $R^2$ | physikalische Konstante |
|---|---|---|---|
| 11 | $CH_3-(CH_2)_2-$ | 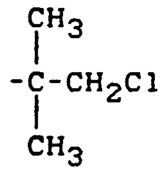 | $n_D^{20}$: 1,5890 |
| 12 | $(CH_3)_2CH-$ | 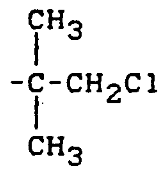 | $n_D^{20}$: 1,5650 |
| 13 | $(CH_3)_3C-$ | 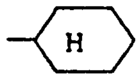 | $^1H$-NMR*): 1,44 (9H), 4,48 (2H) |
| 14 | $(CH_3)_3C-$ | 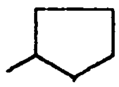 | $^1H$-NMR*): 1,44 (9H), 4,53 (2H) |
| 15 | $(CH_3)_3C-$ | $-C(CH_3)_3$ | Fp. 142 °C |
| 16 | $(CH_3)_3C-$ | 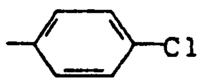 | Fp. 203 °C |
| 17 | $CH_3-(CH_2)_2-$ | $-C(CH_3)_3$ | Fp. 112 °C |
| 18 | $(CH_3)_2CH-$ | $-C(CH_3)_3$ | Fp. 108 °C |
| 19 | 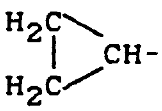 | $-C(CH_3)_3$ | Fp. 151 °C |
| 20 | $(CH_3)_3C-$ | 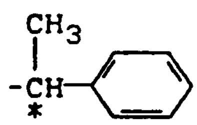 (S-)-Konfig. | $n_D^{20}$: 1,5171 |
| 21 | $(CH_3)_3C-$ | 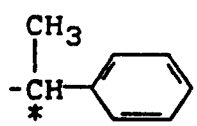 (R+)-Konfig. | $n_D^{20}$: 1,5411 |

| Bsp.-Nr. | $R^1$ | $R^2$ | physikalische Konstante |
|---|---|---|---|
| 22 | $(CH_3)_3C-$ | $CH_3$<br>\|<br>$-C-CH_2-Cl$<br>\|<br>$CH_3$ | Fp. 133 °C |
| 23 | $(CH_3)_3C-$ | $CH_3$<br>\|<br>$-C-C{\equiv}CH$<br>\|<br>$CH_3$ | Fp. 148 °C |
| 24 | $(CH_3)_3C-$ | $CH_3$<br>\|<br>$-C-(CH_2)_3-CH_3$<br>\|<br>$CH_3$ | $n_D^{20}$: 1,4844 |
| 25 | $(CH_3)_3C-$ | $-CH-CH(CH_3)_2$<br>\|<br>$CH_3$ | Fp. 135 °C |
| 26 | $(CH_3)_3C-$ | $CH_3$<br>\|<br>$-C-CH_2F$<br>\|<br>$CH_3$ | Fp. 141 °C |
| 27 | $(CH_3)_3C-$ | 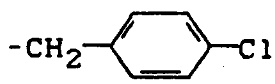 | Fp. 208 °C |
| 28 | $(CH_3)_3C-$ | 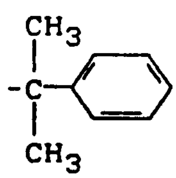 | $n_D^{20}$: 1,5520 |
| 29 | $(CH_3)_3C-$ | 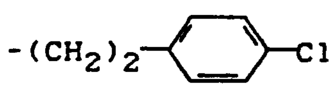 | Fp. 172 °C |
| 30 | $(CH_3)_2CH-$ | $-CH-C_2H_5$<br>\|<br>$CH_3$ | Fp. 83 °C |

| Bsp.-Nr. | $R^1$ | $R^2$ | physikalische Konstante |
|---|---|---|---|
| 31 | $(CH_3)_2CH-$ | 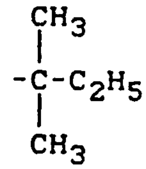 | $n_D^{20}$: 1,4801 |
| 32 | 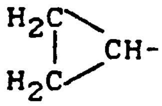 | 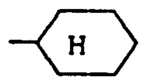 | Fp. 177 °C |
| 33 | 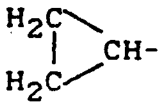 | 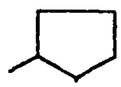 | Fp. 107 °C |
| 34 | $(CH_3)_2CH-$ | 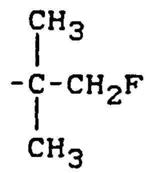 | Fp. 124 °C |
| 35 | $(CH_3)_2CH-$ | 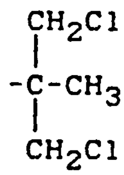 | $^1$H-NMR*): 1,34 (6H), 3,12 (1H), 4,40 (2H) |
| 36 | $(CH_3)_2CH-$ | 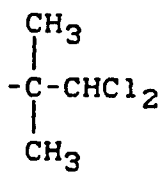 | Fp. 121 °C |
| 37 | $(CH_3)_2CH-$ | $-CH(CH_3)_2$ | Fp. 124 °C |
| 39 | $(CH_3)_2CH-$ | 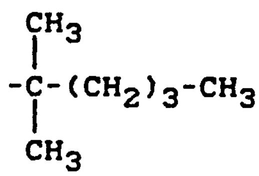 | $n_D^{20}$: 1,4847 |

| Bsp.-Nr. | $R^1$ | $R^2$ | physikalische Konstante |
|---|---|---|---|
| 40 | $(CH_3)_2CH-$ | 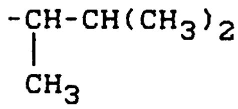 | $n_D^{20}$: 1,4860 |
| 41 | $(CH_3)_2CH-$ | 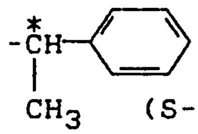 (S-)-Konfig. | $n_D^{20}$: 1,5308 |
| 42 | $(CH_3)_2CH-$ | 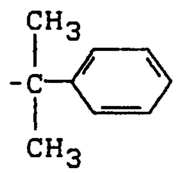 | $n_D^{20}$: 1,5355 |
| 43 | $(CH_3)_2CH-$ | 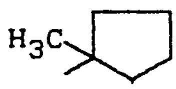 | $n_D^{20}$: 1,5011 |
| 44 | 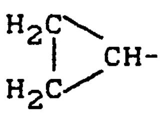 | 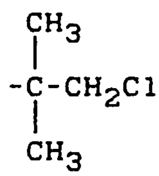 | Fp. 94 °C |
| 45 | 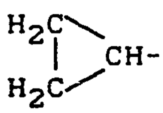 | 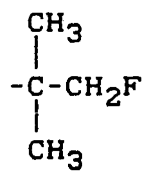 | Fp. 138 °C |
| 46 | $(CH_3)_2CH-$ | 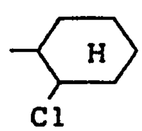 | amorph<br>δ = 1.34 Duplett ($2CH_3$)<br>3.13 Multiplett (1H)<br>3.90 Multiplett (2H)<br>4.48 Singulett ($NH_2$)<br>8.04 Duplett (NH) |

(Fortsetzung)

| Bsp.-Nr. | $R^1$ | $R^2$ | physikalische Konstante |
|---|---|---|---|
| 47 | 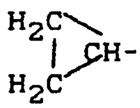 | 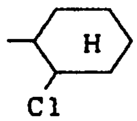 | amorph<br>δ = 1,05 Multiplett (2H, cyclopropyl)<br>1,16 Multiplett (2H, cyclopropyl)<br>2,06 Multiplett (1H, cyclopropyl)<br>3,89 Multiplett (2H)<br>4,55 Singulett ($NH_2$)<br>8,03 Duplett (NH) |
| 48 | $(CH_3)_2CH$ | $-C_4H_9$-n | $n_D^{20}$ : 1,4969 |
| 49 | 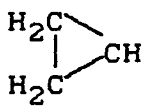 | $-C_4H_9$-n | Fp: 84° C |

*) Die $^1$H-NMR-Spektren wurden in Deuterochloroform ($CDCl_3$) mit Tetramethylsilan (TMS) als innerem Standard aufgenommen. Angegeben ist die chemische Verschiebung als δ-Wert in ppm.

Herstellung der Ausgangsverbindungen

Beispiel III-1

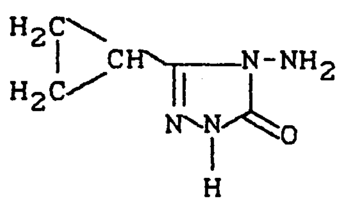

Zu 884 g (17,68 Mol) Hydrazinhydrat gibt man unter Rühren und Eiskühlen portionsweise 1892 g (8,84 Mol) Diphenylcarbonat, so daß die Temperatur der Reaktionsmischung 30 °C nicht übersteigt. Nach beendeter Zugabe rührt man ca. 3 Stunden bei 80 °C, entfernt gebildetes Reaktionswasser dabei im Vakuum, gibt dann 760 g (8,84 Mol) Cyclopropancarbonsäure zu, erhitzt anschließend unter Inertgasatmosphäre im Verlauf von 6 Stunden auf 200 °C und destilliert gleichzeitig frei werdendes Reaktionswasser ab. Nach beendeter Reaktion wird im Vakuum zur Trockne eingeengt, der Rückstand mit 3000 ml siedendem Ethanol extrahiert, filtriert, abgekühlt und der ausgefallene kristalline Niederschlag abgesaugt und getrocknet.

Man erhält 420 g (34 % der Theorie) an 4-Amino-3-cyclopropyl-1,2,4-(1H)-triazolin-5-on vom Schmelzpunkt 181 °C.

In entsprechender Weise erhält man:

Beispiel III-2

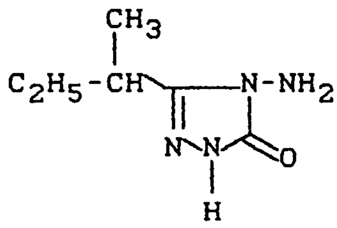

Schmelzpunkt 168 °C

Beispiel III-3

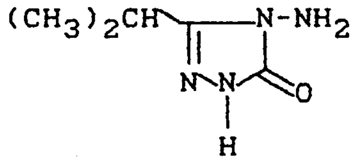

Schmelzpunkt 168 °C

Beispiel III-4

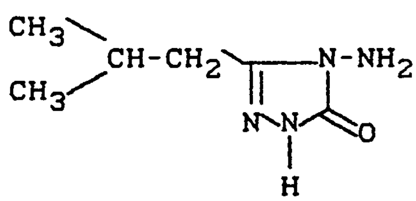

Beispiel III-5

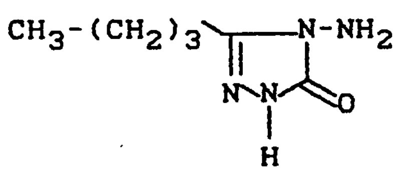

Beispiel (III-6)

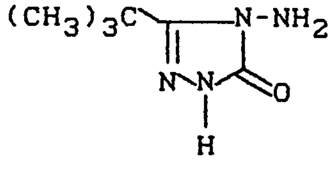

Schmelzpunkt 261 °C

Beispiel VIII-1

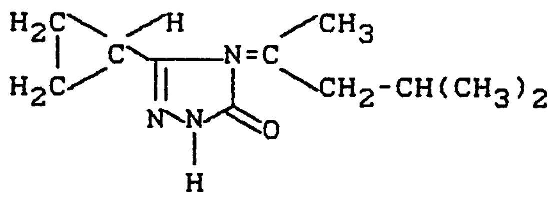

142 g (1,0 Mol) 4-Amino-3-cyclopropyl-1,2,4-(1H)-triazolin-5-on werden in 1000 ml Methylisobutylketon unter Zusatz von 100 mg p-Toluolsulfonsäure solange unter Rückfluß über einen Wasserabscheider erhitzt, bis kein weiteres Reaktionswasser mehr freigesetzt wird. Zur Aufarbeitung wird im Vakuum eingeengt und der Rückstand durch Verreiben mit Petrolether zur Kristallisation gebracht.

Man erhält 66 g (30 % der Theorie) an 4-(4-Methylpent-2-ylidenimino)-3-cyclopropyl-1,2,4-(1H)-triazolin-5-on vom Schmelzpunkt 64 °C.

Anwendungsbeispiele

In den folgenden Anwendungsbeispielen wurden die nachstehend aufgeführten Verbindungen als Vergleichsubstanzen eingesetzt:

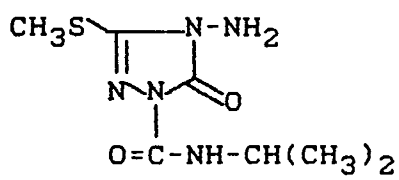

(A)

4-Amino-1-(N-isopropylcarbamoyl)-3-methylthio(1H,4H)-1,2,4-triazolin-5-on

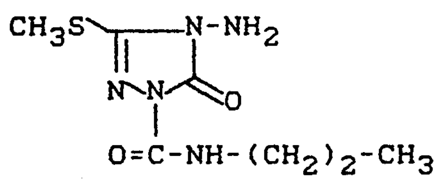

(B)

4-Amino-1-(N-propylcarbamoyl)-3-methylthio-(1H,4H)-1,2,4-triazolin-5-on

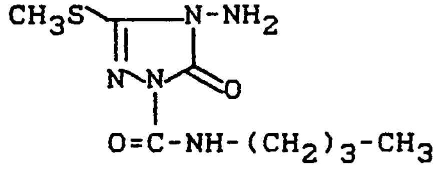

(C)

4 – Amino – 1 – (N – butylcarbamoyl) – 3 – methylthio – (1H,4H) – 1,2,4 – triazolin – 5 – on

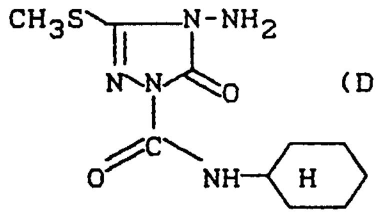 (D)

4 – Amino – 1 – (N – cyclohexylcarbamoyl) – 3 – methylthio – (1H,4H) – 1,2,4 – triazolin – 5 – on
alle bekannt aus JP 52/125 168.

Beispiel A

Pre – emergence – Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Samen der Testpflanzen werden in normalen Boden ausgesät und nach 24 Stunden mit der Wirkstoff – zubereitung begossen. Dabei hält man die Wassermenge pro Flächeneinheit zweckmäßigerweise konstant. Die Wirkstoffkonzentration in der Zubereitung spielt keine Rolle, entscheidend ist nur die Aufwandmenge des Wirkstoffs pro Flächeneinheit. Nach drei Wochen wird der Schädigungsgrad der Pflanzen bonitiert in % Schädigung im Vergleich zur Entwicklung der unbehandelten Kontrolle. Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß folgender Herstellungsbeispiele: (1), (10), (18), (30), (31), (34), (37) und (40).

Beispiel B

Post – emergence – Test

Lösungsmittel: 5 Gewichtsteile Aceton
Emulgator: 1 Gewichtsteil Alkylarylpolyglykolether

Zur Herstellung einer zweckmäßigen Wirkstoffzubereitung vermischt man 1 Gewichtsteil Wirkstoff mit der angegebenen Menge Lösungsmittel, gibt die angegebene Menge Emulgator zu und verdünnt das Konzentrat mit Wasser auf die gewünschte Konzentration.

Mit der Wirkstoffzubereitung spritzt man Testpflanzen, welche eine Höhe von 5 – 15 cm haben so, daß die jeweils gewünschten Wirkstoffmengen pro Flächeneinheit ausgebracht werden. Die Konzentration der Spritzbrühe wird so gewählt, daß in 2000 l Wasser/ha die jeweils gewünschten Wirkstoffmengen ausge – bracht werden. Nach drei Wochen wird der Schädigungsgrad der Pflanzen bonitiert in % Schädigung im Vergleich zur Entwicklung der unbehandelten Kontrolle.
Es bedeuten:

0 % = keine Wirkung (wie unbehandelte Kontrolle)
100 % = totale Vernichtung

Eine deutliche Überlegenheit in der Wirksamkeit bei vergleichbarer Nutzpflanzenselektivität gegenüber dem Stand der Technik zeigen in diesem Test z.B. die Verbindungen gemäß folgender Herstellungsbeispiele: (12), (18), (19), (30), (34), (36), (37), (39), und (40).

## Patentansprüche

**1.** Substituierte Triazolinone der allgemeinen Formel (I),

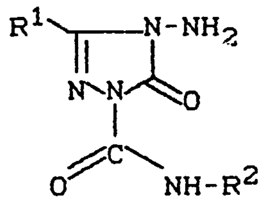

(I)

in welcher

$R^1$ für Alkyl mit 3 bis 4 Kohlenstoffatomen oder für Cyclopropyl steht und

$R^2$ für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl, Aryl, Aralkyloxy oder Aryloxy, für Alkoxy, Alkenyloxy oder Alkinyloxy steht.

**2.** Substituierte Triazolinone der Formel (I) gemäß Anspruch 1, in welcher

$R^1$ für n-Propyl, i-Propyl, n-Butyl, i-Butyl, s-Butyl,t-Butyl oder Cyclopropyl steht und

$R^2$ für Wasserstoff, für jeweils geradkettiges oder verzweigtes Alkyl mit 1 bis 18 Kohlenstoffatomen, Alkenyl mit 2 bis 8 Kohlenstoffatomen, Alkinyl mit 2 bis 8 Kohlenstoffatomen, Halogenalkyl mit 1 bis 8 Kohlenstoffatomen und 1 bis 17 gleichen oder verschiedenen Halogenatomen, Halogenalkenyl oder Halogenalkinyl mit jeweils 2 bis 8 Kohlenstoffatomen und 1 bis 15 bzw. 13 gleichen oder verschiedenen Halogenatomen, Cyanalkyl mit 1 bis 8 Kohlenstoffatomen, Hydroxyalkyl mit 1 bis 8 Kohlenstoffatomen und 1 bis 6 Hydroxygruppen, Alkoxyalkyl, Alkoxycarbonylalkyl oder Alkoxycarbonylalkenyl mit jeweils bis zu 6 Kohlenstoffatomen in den einzelnen Alkyl- bzw. Alkenylteilen, Alkylaminoalkyl oder Dialkylaminoalkyl mit jeweils 1 bis 6 Kohlenstoffatomen in den einzelnen Alkylteilen, für Cycloalkyl mit 11 Kohlenstoffatomen oder für jeweils gegebenenfalls einfach oder mehrfach gleich oder verschieden substituiertes Cycloalkyl, Cycloalkylalkyl, Cycloalkenyl oder Cycloalkenylalkyl mit jeweils 3 bis 8 Kohlenstoffatomen im Cycloalkyl- bzw. Cycloalkenylteil und gegebenenfalls 1 bis 6 Kohlenstoffatomen im Alkylteil steht, wobei als Substituenten jeweils infrage kommen: Halogen, Cyano sowie jeweils geradkettiges oder verzweigtes Alkyl oder Halogenalkyl mit jeweils 1 bis 4 Kohlenstoffatomen und gegebenenfalls 1 bis 9 gleichen oder verschiedenene Halogenatomen oder geradkettiges oder verzweigtes Halogenalkenyl mit bis zu 4 Kohlenstoffatomen und 1 bis 5 gleichen oder verschiedenen Halogenatomen oder jeweils zweifach verknüpftes Alkandiyl oder Alkendiyl mit jeweils bis zu 4 Kohlenstoffatomen; $R^2$ außerdem für im Heterocyclylteil gegebenenfalls einfach oder mehrfach, gleich oder verchieden substituiertes Heterocyclylalkyl mit 1 bis 6 Kohlenstoffatomen im geradkettigen oder verzweigten Alkylteil und 1 bis 9 Kohlenstoffatomen sowie 1 bis 3 Heteroatomen - insbesondere Stickstoff, Sauerstoff und/oder Schwefel - im Heterocyclylteil steht, wobei als Substituenten infrage kommen: Halogen, Cyano, Nitro, sowie jeweils geradkettiges oder verzweigtes Alkyl, Alkoxy, Alkylthio, Halogenalkyl, Halogenalkoxy, Halogenalkylthio oder Alkoxycarbonyl mit jeweils 1 bis 5 Kohlenstoffatomen und gegebenenfalls 1 bis 9 gleichen oder verschiedenen Halogenatomen; $R^2$ außerdem für jeweils geradkettiges oder verzweigtes Alkoxy mit 1 bis 8 Kohlenstoffatomen, Alkenyloxy mit 2 bis 8 Kohlenstoffatomen oder Alkinyloxy mit 2 bis 8 Kohlenstoffatomen und schließlich für jeweils gegebenenfalls einfach oder mehrfach, gleich oder verschieden substituiertes Aralkyl, Aroyl, Aryl, Aralkyloxy oder Aryloxy mit jeweils 6 bis 10 Kohlenstoffatomen im Arylteil und gegebenenfalls 1 bis 8 Kohlenstoffatomen im geradkettigen oder verzweigten Alkylteil steht, wobei als Alkylsubstituenten gegebenenfalls Halogen und Cyano infrage kommen und wobei als Arylsubstituenten jeweils infrage kommen: Halogen, Cyano, Nitro, Hydroxy, jeweils geradkettiges oder verzweigtes Alkyl, Alkoxy, Alkylthio, Halogenalkyl, Halogenalkoxy, Halogenalkylthio, Alkylsulfinyl, Alkylsulfonyl, Halogenalkylsulfinyl, Halogenalkylsulfonyl, Alkanoyl oder Alkoxycarbonyl mit jeweils 1 bis 6 Kohlenstoffatomen im Alkylteil und gegebenenfalls 1 bis 9 gleichen oder verschiedenen Halogenatomen, Cycloalkyl mit 3 bis 6 Kohlenstoffatomen und Phenoxy; oder $R^2$ für Benzyl mit im Phenylteil ankondensierter $-O-CH_2-O-$ Gruppe steht.

**3.** Verfahren zur Herstellung von substituierten Triazolinonen der allgemeinen Formel (I),

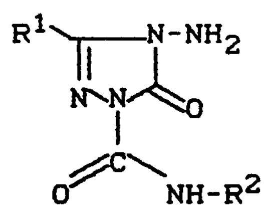

(I)

in welcher

$R^1$ für Alkyl mit 3 bis 4 Kohlenstoffatomen oder für Cyclopropyl steht und

$R^2$ für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl, Aryl, Aralkyloxy oder Aryloxy, für Alkoxy, Alkenyloxy oder Alkinyloxy steht,

dadurch gekennzeichnet, daß man

(a) Hydrazone der Formel (II),

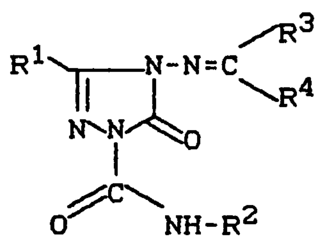

(II)

in welcher

$R^1$ und $R^2$ die oben angegebene Bedeutung haben und

$R^3$ und $R^4$ unabhängig voneinander jeweils für Wasserstoff, Alkyl, Aralkyl oder Aryl stehen,

mit einer Säure gegebenenfalls in Gegenwart eines Verdünnungsmittels umsetzt,

oder daß man

(b) 1H-Triazolinone der Formel (III),

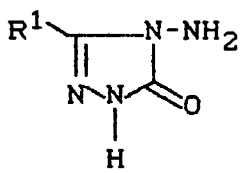

(III)

in welcher

$R^1$ die oben angegebene Bedeutung hat,

mit Isocyanaten der Formel (IV),

$R^2-N=C=O$ (IV)

in welcher
$R^2$ die oben angegebene Bedeutung hat,
gegebenenfalls in Gegenwart eines Verdünnungsmittels und gegebenenfalls in Gegenwart eines Reaktionshilfsmittels umsetzt,
oder daß man
(c) Triazolinone der Formel (V),

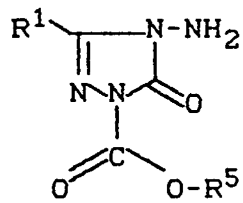

(V)

in welcher
$R^1$ die oben angegebene Bedeutung hat, und
$R^5$ für Alkyl, Aryl oder Arylalkyl steht,
mit Aminen der Formel (VI),

$R^2 - NH_2$ (VI)

in welcher
$R^2$ die oben angegebene Bedeutung hat,
gegebenenfalls in Gegenwart eines Verdünnungsmittels und gegebenenfalls in Gegenwart eines Reaktionshilfsmittels umsetzt,
oder daß man
(d) 1H – Triazolinone der Formel (III),

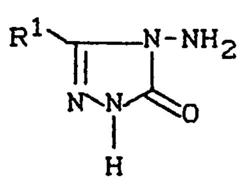

(III)

in welcher
$R^1$ die oben angegebene Bedeutung hat,
mit Urethanen der Formel (VII),

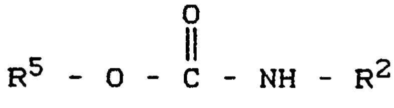

(VII)

in welcher
$R^2$ die oben angegebene Bedeutung hat und
$R^5$ für Alkyl, Aryl oder Arylalkyl steht,
gegebenenfalls in Gegenwart eines Verdünnungsmittels und gegebenenfalls in Gegenwart eines Reaktionshilfsmittels umsetzt.

**4.** Herbizide Mittel, gekennzeichnet durch einen Gehalt an mindestens einem substituierten Triazolinon der Formel (I) gemäß den Ansprüchen 1 bis 3.

**5.** Verfahren zur Bekämpfung von Unkräutern, dadurch gekennzeichnet, daß man substituierte Triazolinone der Formel (I) gemäß Ansprüchen 1 bis 3 auf die Unkräuter und/oder ihren Lebensraum einwirken läßt.

**6.** Verwendung von substituierten Triazolinonen der Formel (I) gemäß den Ansprüchen 1 bis 3 zur Bekämpfung von Unkräutern.

**7.** Verfahren zur Herstellung von herbiziden Mitteln, dadurch gekennzeichnet, daß man substituierte Triazolinone der Formel (I) gemäß den Ansprüchen 1 bis 3 mit Streckmittel und/oder oberflächenaktiven Substanzen vermischt.

**8.** Hydrazone der Formel (II),

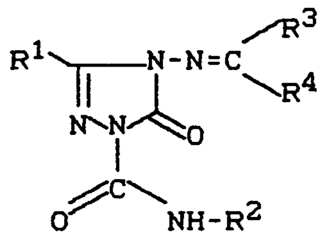

(II)

in welcher

| | |
|---|---|
| $R^1$ | für Alkyl mit 3 bis 4 Kohlenstoffatomen oder für Cyclopropyl steht und |
| $R^2$ | für Wasserstoff, Alkyl, Alkenyl, Alkinyl, Halogenalkyl, Halogenalkenyl, Halogenalkinyl, Cyanalkyl, Hydroxyalkyl, Alkoxyalkyl, Alkoxycarbonylalkyl, Alkoxycarbonylalkenyl, Alkylaminoalkyl, Dialkylaminoalkyl, für jeweils gegebenenfalls substituiertes Cycloalkyl, Cycloalkylalkyl, Cycloalkenyl oder Cycloalkenylalkyl, für gegebenenfalls substituiertes Heterocyclylalkyl, für jeweils gegebenenfalls substituiertes Aralkyl, Aroyl, Aryl, Aralkyloxy oder Aryloxy, für Alkoxy, Alkenyloxy oder Alkinyloxy steht und |
| $R^3$ und $R^4$ | unabhängig voneinander jeweils für Wasserstoff, Alkyl, Aralkyl oder Aryl stehen. |

**9.** 1-unsubstituierte 4-Aminotriazolinone der Formel (IIIa)

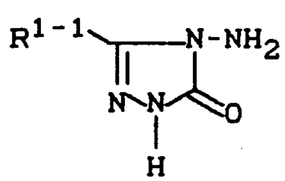

(IIIa)

in welcher

$R^{1-1}$ für n-Butyl, i-Butyl, s-Butyl oder Cyclopropyl steht.

**10.** Triazolinone der Formel (XI)

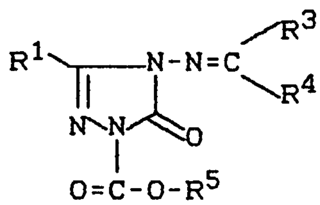

(XI)

in welcher

| | |
|---|---|
| $R^1$ | für Alkyl mit 3 bis 4 Kohlenstoffatomen oder für Cyclopropyl steht, |
| $R^3$ und $R^4$ | unabhängig voneinander für Wasserstoff, Alkyl, Aralkyl oder Aryl stehen und |

$R^5$ für Alkyl, Aryl oder Arylalkyl steht.

**Claims**

**1.** Substituted triazolinones of the general formula (I)

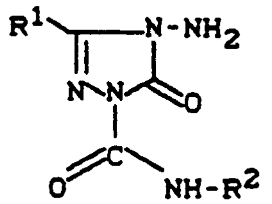

(I)

in which

$R^1$ represents alkyl having 3 to 4 carbon atoms, or represents cyclopropyl, and

$R^2$ represents hydrogen, alkyl, alkenyl, alkinyl, halogenoalkyl, halogenoalkenyl, halogenoalkinyl, cyanoalkyl, hydroxyalkyl, alkoxyalkyl, alkoxycarbonylalkyl, alkoxycarbonylalkenyl, alkylaminoalkyl or dialkylaminoalkyl, or represents cycloalkyl, cycloalkylalkyl, cycloalkenyl or cycloalkenylalkyl, each of which is optionally substituted, or represents optionally substituted heterocyclylalkyl, or represents aralkyl, aroyl, aryl, aralkyloxy or aryloxy, each of which is optionally substituted, or represents alkoxy, alkenyloxy or alkinyloxy.

**2.** Substituted triazolinones of the formula (I) according to Claim 1, in which

$R^1$ represents n-propyl, i-propyl, n-butyl, i-butyl, s-butyl, t-butyl or cyclopropyl and

$R^2$ represents hydrogen, or represents in each case straight-chain or branched alkyl having 1 to 18 carbon atoms, alkenyl having 2 to 8 carbon atoms, alkinyl having 2 to 8 carbon atoms, halogenoalkyl having 1 to 8 carbon atoms and 1 to 17 identical or different halogen atoms, halogenoalkenyl or halogenoalkinyl, each of which has 2 to 8 carbon atoms and 1 to 15 or 13 identical or different halogen atoms, cyanoalkyl having 1 to 8 carbon atoms, hydroxyalkyl having 1 to 8 carbon atoms and 1 to 6 hydroxyl groups, alkoxyalkyl, alkoxycarbonylalkyl or alkoxycarbonylalkenyl, each having up to 6 carbon atoms in the individual alkyl or alkenyl moieties, or alkylaminoalkyl or dialkylaminoalkyl, each having 1 to 6 carbon atoms in the individual alkylmoieties, or represents cycloalkyl having 11 carbon atoms, or represents cycloalkyl, cycloalkylalkyl, cycloalkenyl or cycloalkenylalkyl, each of which has 3 to 8 carbon atoms in the cycloalkyl or cycloalkenyl moiety and if appropriate 1 to 6 carbon atoms in the alkyl moiety, and each of which is optionally monosubstituted or polysubstituted by identical or different substituents, suitable substituents in each case being: halogen, cyano as well as in each case straight-chain or branched alkyl or halogenoalkyl, each of which has 1 to 4 carbon atoms and if appropriate 1 to 9 identical or different halogen atoms, or straight-chain or branched halogenoalkenyl having up to 4 carbon atoms and 1 to 5 identical or different halogen atoms, or in each case double-linked alkanediyl or alkenediyl, in each case having up to 4 carbon atoms; $R^2$ furthermore represents heterocyclylalkyl which has 1 to 6 carbon atoms in the straight-chain or branched alkyl moiety and 1 to 9 carbon atoms and 1 to 3 hetero atoms – in particular nitrogen, oxygen and/or sulphur – in the heterocyclyl moiety and which is optionally monosubstituted or polysubstituted in the heterocyclyl moiety by identical or different substituents, suitable substituents being: halogen, cyano, nitro, and in each case straight-chain or branched alkyl, alkoxy, alkylthio, halogenoalkyl, halogenoalkoxy, halogenoalkylthio or alkoxycarbonyl, each of which has 1 to 5 carbon atoms and if appropriate 1 to 9 identical or different halogen atoms; $R^2$ furthermore represents in each case straight-chain or branched alkoxy having 1 to 8 carbon atoms, alkenyloxy having 2 to 8 carbon atoms or alkinyloxy having 2 to 8 carbon atoms, and finally represents aralkyl, aroyl, aryl, aralkyloxy or aryloxy, each of which has 6 to 10 carbon atoms in the aryl moiety and if appropriate 1 to 8 carbon atoms in the straight-chain or branched alkyl moiety and each of which is optionally monosubstituted or polysubstituted by identical or different substituents, suitable alkyl substituents, where appropriate, being halogen and cyano, and suitable aryl substituents in each case being: halogen, cyano, nitro, hydroxyl, in each case straight-chain or branched

alkyl, alkoxy, alkylthio, halogenoalkyl, halogenoalkoxy, halogenoalkylthio, alkylsulphinyl, alkylsulphonyl, halogenoalkylsulphinyl, halogenoalkylsulphonyl, alkanoyl or alkoxycarbonyl, each of which has 1 to 6 carbon atoms in the alkyl moiety and if appropriate 1 to 9 identical or different halogen atoms, or cycloalkyl having 3 to 6 carbon atoms and phenoxy; or $R^2$ represents benzyl with an $-O-CH_2-O-$ group fused to the phenyl moiety.

**3.** Process for the preparation of the substituted triazolinones of the general formula (I)

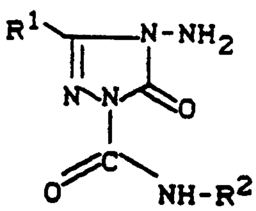

(I)

in which

$R^1$ represents alkyl having 3 to 4 carbon atoms, or represents cyclopropyl, and

$R^2$ represents hydrogen, alkyl, alkenyl, alkinyl, halogenoalkyl, halogenoalkenyl, halogenoalkinyl, cyanoalkyl, hydroxyalkyl, alkoxyalkyl, alkoxycarbonylalkyl, alkoxycarbonylalkenyl, alkylaminoalkyl or dialkylaminoalkyl, or represents cycloalkyl, cycloalkylalkyl, cycloalkenyl or cycloalkenylalkyl, each of which is optionally substituted, or represents optionally substituted heterocyclylalkyl, or represents aralkyl, aroyl, aryl, aralkyloxy or aryloxy, each of which is optionally substituted, or represents alkoxy, alkenyloxy or alkinyloxy,

characterized in that

(a) hydrazones of the formula (II)

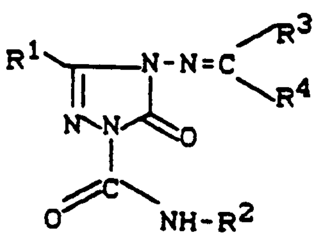

(II)

in which

$R^1$ and $R^2$ have the abovementioned meaning and

$R^3$ and $R^4$ independently of one another each represent hydrogen, alkyl, aralkyl or aryl

are reacted with an acid, if appropriate in the presence of a diluent,

or in that

(b) 1H-triazolinones of the formula (III)

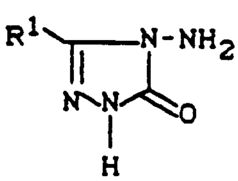

(III)

in which

$R^1$ has the abovementioned meaning,

are reacted with isocyanates of the formula (IV)

$R^2-N=C=O$ (IV)

in which

$R^2$ has the abovementioned meaning,

if appropriate in the presence of a diluent and if appropriate in the presence of a reaction auxiliary, or in that

(c) triazolinones of the formula (V)

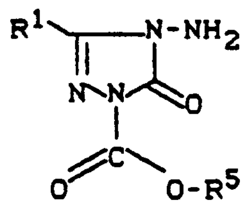

(V)

in which

$R^1$ has the abovementioned meaning, and

$R^5$ represents alkyl, aryl or arylalkyl,

are reacted with amines of the formula (VI)

$R^2 - NH_2$ (VI)

in which

$R^2$ has the abovementioned meaning,

if appropriate in the presence of a diluent and if appropriate in the presence of a reaction auxiliary, or in that

(d) 1H-triazolinones of the formula (III)

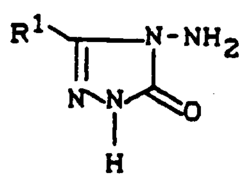

(III)

in which

$R^1$ has the abovementioned meaning,

are reacted with urethanes of the formula (VII)

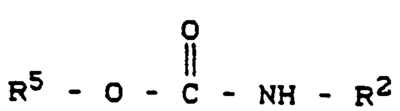

(VII)

in which

$R^2$ has the abovementioned meaning and

$R^5$ represents alkyl, aryl or arylalkyl,

if appropriate in the presence of a diluent and if appropriate in the presence of a reaction auxiliary.

**4.** Herbicidal agents, characterized in that they contain at least one substituted triazolinone of the formula (I) according to Claims 1 to 3.

**5.** Method of combating weeds, characterized in that substituted triazolinones of the formula (I) according to Claims 1 to 3 are allowed to act on the weeds and/or their environment.

**6.** Use of substituted triazolinones of the formula (I) according to Claims 1 to 3 for combating weeds.

**7.** Process for the preparation of herbicidal agents, characterized in that substituted triazolinones of the formula (I) according to Claims 1 to 3 are mixed with an extender and/or surface-active substances.

**8.** Hydrazones of the formula (II)

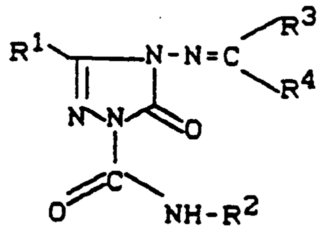

(II)

in which

| | |
|---|---|
| $R^1$ | represents alkyl having 3 to 4 carbon atoms, or represents cyclopropyl, and |
| $R^2$ | represents hydrogen, alkyl, alkenyl, alkinyl, halo-genoalkyl, halogenoalkenyl, halogenoalkinyl, cyanoalkyl, hydroxyalkyl, alkoxyalkyl, alkoxycarbonylalkyl, alkoxycarbonylalkenyl, alkylaminoalkyl or dialkylaminoalkyl, or represents cycloalkyl, cycloalkylalkyl, cycloalkenyl or cycloalkenylalkyl, each of which is optionally substituted, or represents optionally substituted heterocyclylalkyl, or represents aralkyl, aroyl, aryl, aralkyloxy or aryloxy, each of which is optionally substituted, or represents alkoxy, alkenyloxy or alkinyloxy and |
| $R^3$ and $R^4$ | independently of one another each represent hydrogen, alkyl, aralkyl or aryl. |

**9.** 1-Unsubstituted 4-aminotriazolinones of the formula (IIIa)

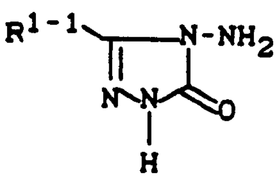

(IIIa)

in which

$R^{1-1}$ represents n-butyl, i-butyl, s-butyl or cyclopropyl.

**10.** Triazolinones of the formula (XI)

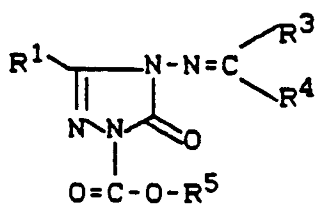

(XI)

in which

| | |
|---|---|
| $R^1$ | represents alkyl having 3 to 4 carbon atoms or represents cyclopropyl, |
| $R^3$ and $R^4$ | independently of one another represent hydrogen, alkyl, aralkyl or aryl and |
| $R^5$ | represents alkyl, aryl or arylalkyl. |

**Revendications**

**1.** Triazolinones substituées de formule générale I

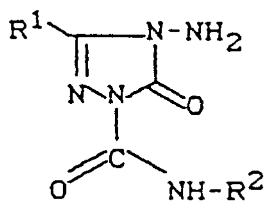

(I)

dans laquelle
$R^1$ représente un groupe alkyle en $C_3-C_4$ ou un groupe cyclopropyle et
$R^2$ représente l'hydrogène, un groupe alkyle, alcényle, alcynyle, halogénoalkyle, halogénoalcényle, halogénoalcynyle, cyanalkyle, hydroxyalkyle, alcoxyalkyle, alcoxycarbonylalkyle, alcoxycarbonylalcényle, alkylaminoalkyle, dialkylaminoalkyle, un groupe cycloalkyle, cycloalkylalkyle, cycloalcényle ou cycloalcénylalkyle, chacun d'eux éventuellement substitué, un groupe hétérocyclylalkyle éventuellement substitué, un groupe aralkyle, aroyle, aryle, aralkyloxy ou aryloxy, chacun d'eux éventuellement substitué, un groupe alcoxy, alcényloxy ou alcynyloxy.

**2.** Triazolinones substituées de formule I de la revendication 1 dans laquelle
$R^1$ représente un groupe n-propyle, isopropyle, n-butyle, isobutyle, sec-butyle, tert-butyle ou cyclopropyle et
$R^2$ représente l'hydrogène, un groupe alkyle en $C_1-C_{18}$, alcényle en $C_2-C_8$, alcynyle en $C_2-C_8$, halogénoalkyle contenant 1 à 8 atomes de carbone et 1 à 17 atomes d'halogènes identiques ou différents, halogénoalcényle ou halogénoalcynyle contenant chacun 2 à 8 atomes de carbone et respectivement 1 à 15 et 1 à 13 atomes d'halogènes identiques ou différents, un groupe cyanalkyle en $C_1-C_8$, hydroxyalkyle contenant 1 à 8 atomes de carbone et 1 à 6 groupes hydroxy, chacun d'eux à chaîne droite ou ramifiée, un groupe alcoxyalkyle, alcoxycarbonylalkyle ou alcoxycarbonylalcényle contenant chacun jusqu'à 6 atomes de carbone dans les diverses parties alkyle et alcényle, un groupe alkylaminoalkyle ou dialkylaminoalkyle contenant chacun 1 à 6 atomes de carbone dans les diverses parties alkyle, un groupe cycloalkyle à 11 atomes de carbone ou un groupe cycloalkyle, cycloalkylalkyle, cycloalcényle ou cycloalcénylalkyle portant chacun le cas échéant un ou plusieurs substituants identiques ou différents et contenant chacun 3 à 8 atomes de carbone dans la partie cycloalkyle ou cycloalcényle et le cas échéant 1 à 6 atomes de carbone dans la partie alkyle, les substituants en question étant : des halogènes, des groupes cyano et des groupes alkyle ou halogénoalkyle, chacun d'eux à chaîne droite ou ramifiée, contenant chacun 1 à 4 atomes de carbone et le cas échéant 1 à 9 atomes d'halogènes identiques ou différents, ou des groupes halogénoalcényle à chaîne droite ou ramifiée contenant jusqu'à 4 atomes de carbone et 1 à 5 atomes d'halogènes identiques ou différents ou des groupes alcane-diyle ou alcène-diyle fixés chacun par deux liaisons et contenant chacun jusqu'à 4 atomes de carbone ; $R^2$ peut en outre représenter un groupe hétérocyclylalkyle portant éventuellement un ou plusieurs substituants identiques ou différents dans la partie hétérocyclyle et contenant 1 à 6 atomes de carbone dans la partie alkyle à chaîne droite ou ramifiée et 1 à 9 atomes de carbone, avec 1 à 3 hétéroatomes - en particulier d'azote, d'oxygène et/ou de soufre - dans la partie hétérocyclyle, les substituants en question étant : des halogènes, des groupes cyano, nitro, des groupes alkyle, alcoxy, alkylthio, halogénoalkyle, halogénoalcoxy, halogénoalkylthio ou alcoxycarbonyle, chacun d'eux à chaîne droite ou ramifiée et contenant chacun 1 à 5 atomes de carbone et le cas échéant 1 à 9 atomes d'halogènes identiques ou différents ; $R^2$ peut en outre représenter un groupe alcoxy en $C_1-C_8$, alcényloxy en $C_2-C_8$ ou alcynyloxy en $C_2-C_8$, chacun d'eux à chaîne droite ou ramifiée, et, finalement, un groupe aralkyle, aroyle, aryle, aralkyloxy ou aryloxy portant chacun le cas échéant un ou plusieurs substituants identiques ou différents et contenant chacun 6 à 10 atomes de carbone dans la partie aryle et le cas échéant 1 à 8 atomes de carbone dans la partie alkyle à chaîne droite ou ramifiée, les substituants de la partie alkyle étant éventuellement des halogènes et des groupes cyano et les substituants de la partie aryle étant : des halogènes, des groupes cyano, nitro, hydroxy, des groupes alkyle, alcoxy, alkythio, halogénoalkyle, halogénoalcoxy, halogénoalkylthio, alkylsulfinyle, alkylsulfonyle, halogénoalkylsulfinyle, halogénoalkylsulfonyle, alcanoyle ou alcoxycarbonyle, chacun d'eux à chaîne droite ou ramifiée et contenant chacun 1 à 6 atomes de carbone dans la

partie alkyle et le cas échéant 1 à 9 atomes d'halogènes identiques ou différents, des groupes cycloalkyle en $C_3-C_6$ et phénoxy ; ou bien $R^2$ représente un groupe benzyle avec un groupe $-O-CH_2-O-$ condensé sur la partie phényle.

**3.** Procédé de préparation des triazolinones substituées de formule générale I

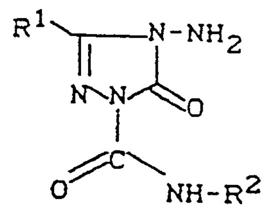

(I)

dans laquelle
$R^1$ représente un groupe alkyle en $C_3-C_4$ ou un groupe cyclopropyle et
$R^2$ représente l'hydrogène, un groupe alkyle, alcényle, alcynyle, halogénoalkyle, halogénoalcényle, halogénoalcynyle, cyanalkyle, hydroxyalkyle, alcoxyalkyle, alcoxycarbonylalkyle, alcoxycarbonylalcényle, alkylaminoalkyle, dialkylaminoalkyle, un groupe cycloalkyle, cycloalkylalkyle, cycloalcényle ou cycloalcénylalkyle, chacun d'eux éventuellement substitué, un groupe hétérocyclylalkyle éventuellement substitué, un groupe aralkyle, aroyle, aryle, aralkyloxy ou aryloxy, chacun d'eux éventuellement substitué, un groupe alcoxy, alcényloxy ou alcynyloxy,
caractérisé en ce que
(a) on fait réagir des hydrazones de formule II

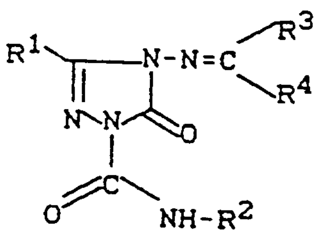

(II)

dans laquelle
$R^1$ et $R^2$ ont les significations indiquées ci-dessus et
$R^3$ et $R^4$ représentent chacun, indépendamment l'un de l'autre, l'hydrogène, un groupe alkyle, aralkyle ou aryle,
avec un acide, éventuellement en présence d'un diluant,
ou bien
(b) on fait réagir des 1H-triazolinones de formule III

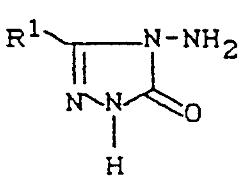

(III)

dans laquelle $R^1$ a les significations indiquées ci-dessus,
avec des isocyanates de formule IV

$R^2 - N = C = O$ (IV)

dans laquelle $R^2$ a les significations indiquées ci-dessus,
éventuellement en présence d'un diluant et éventuellement en présence d'un produit auxiliaire de réaction,

ou bien
(c) on fait réagir des triazolinones de formule V

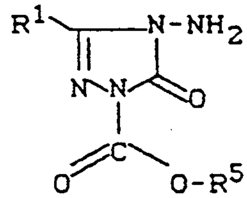

(V)

dans laquelle
$R^1$ a les significations indiquées ci-dessus et
$R^5$ représente un groupe alkyle, aryle ou arylalkyle,
avec des amines de formule VI

$R^2-NH_2$ (VI)

dans laquelle $R^2$ a les significations indiquées ci-dessus,
éventuellement en présence d'un diluant et éventuellement en présence d'un produit auxiliaire de réaction,
ou bien
(d) on fait réagir des 1H-triazolinones de formule III

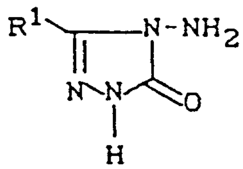

(III)

dans laquelle $R^1$ a les significations indiquées ci-dessus,
avec des uréthannes de formule VII

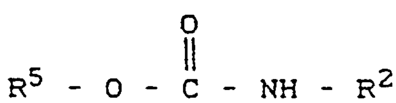

(VII)

dans laquelle
$R^2$ a les significations indiquées ci-dessus et
$R^5$ représente un groupe alkyle, aryle ou arylalkyle,
éventuellement en présence d'un diluant et éventuellement en présence d'un produit auxiliaire de réaction.

**4.** Produits herbicides caractérisés en ce qu'ils contiennent au moins une triazolinone substituée de formule I selon les revendications 1 à 3.

**5.** Procédé pour combattre les végétaux adventices, caractérisé en ce que l'on fait réagir sur les végétaux adventices et/ou leur habitat des triazolinones substituées de formule I selon les revendications 1 à 3.

**6.** Utilisation des triazolinones substituées de formule I selon les revendications 1 à 3 pour la lutte contre les végétaux adventices.

7. Procédé de préparation de produits herbicides, caractérisé en ce que l'on mélange des triazolinones substituées de formule I selon les revendications 1 à 3 avec des diluants et/ou des agents tensio-actifs.

8. Hydrazones de formule II

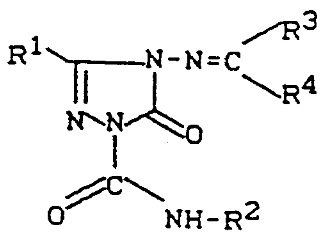

(II)

dans laquelle
$R^1$ représente un groupe alkyle en $C_3-C_4$ ou un groupe cyclopropyle et
$R^2$ représente l'hydrogène, un groupe alkyle, alcényle, alcynyle, halogénoalkyle, halogénoalcényle, halogénoalcynyle, cyanalkyle, hydroxyalkyLe, alcoxyalkyle, alcoxycarbonylalkyle, alcoxycarbonylalcényle, alkylaminoalkyle, dialkylaminoalkyle, un groupe cycloalkyle, cycloalkylalkyle, cycloalcényle ou cycloalcénylalkyle, chacun d'eux éventuellement substitué, un groupe hétérocyclylalkyle éventuellement substitué, un groupe aralkyle, aroyle, aryle, aralkyloxy ou aryloxy, chacun d'eux éventuellement substitué, un groupe alcoxy, alcényloxy ou alcynyloxy et
$R^3$ et $R^4$ représentent chacun, indépendamment l'un de l'autre, l'hydrogène, un groupe alkyle, aralkyle ou aryle.

9. 4-aminotriazolinones non substituées en position 1, de formule IIIa

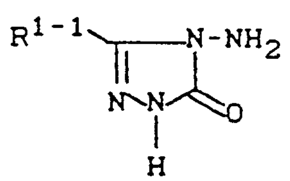

(IIIa)

dans laquelle
$R^{1-1}$ représente un groupe n-butyle, iso-butyle, sec-butyle ou cyclopropyle.

10. Triazolinones de formule XI

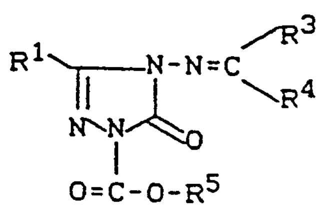

(XI)

dans laquelle
$R^1$ représente un groupe alkyle en $C_3-C_4$ ou cyclopropyle,
$R^3$ et $R^4$ représentent chacun, indépendamment l'un de l'autre, l'hydrogène, un groupe alkyle, aralkyle ou aryle et
$R^5$ représente un groupe alkyle, aryle ou arylalkyle.